# EUROPEAN PATENT SPECIFICATION

(11) **EP 1 510 130 B1**
(45) Date of publication and mention of the grant of the patent: **03.06.2015**
(21) Application number: 04019169.4
(22) Date of filing: 12.08.2004
(51) Int. Cl.: A01N 47/28, A01N 47/44, A01N 41/04, A01N 37/44, A01N 61/00, B01D 39/14, F24F 3/16, A62B 23/00, A61L 2/00, A61L 9/00

(54) **Virus inactivating method and use of inactivating agent in filter set and in air conditioners**
Verfahren zur Inaktivierung von Viren und Verwendung in einem Filtersatz und in einer Klimaanlage
Procédé pour l'inactivation des virus et utilisation dans une ensemble de filtre et dans un conditionneur d'air

(30) Priority: 19.08.2003 JP 2003207883; 07.10.2003 JP 2003348670
(43) Date of publication of application: 02.03.2005
(73) Proprietor: MITSUBISHI HEAVY INDUSTRIES, LTD., Tokyo 108-8215 (JP)
(72) Inventor: Nakajima, Yuji, Yokohama, Kanagawa-ken (JP); Hashitsume, Katsuhiro, Nagoya, Aichi-ken (JP); Tanaka, Daisuke, Nagoya, Aichiken (JP)
(74) Representative: Hoffmann Eitle

(56) References cited:
- EP-A- 0 958 851
- EP-A- 1 460 348
- EP-A1- 0 870 833
- WO-A-01/12296
- WO-A-94/00166
- WO-A-99/59525
- US-A- 4 876 070
- DATABASE WPI Section Ch, Week 199012 Derwent Publications Ltd., London, GB; Class A88, AN 1990-087667 XP002307895 & JP 02 041166 A (SHIN NIPPON MUSEN) 9 February 1990 (1990-02-09)
- DATABASE WPI Section Ch, Week 198517 Derwent Publications Ltd., London, GB; Class A96, AN 1985-103142 XP002307896 & JP 60 049795 A (KANAI H) 19 March 1985 (1985-03-19)
- DATABASE CAPLUS [Online] CHEMICAL ABSTRACTS SERVICE, COLUMBUS, OHIO, US; A. DENYS: "Effect of guanidine hydrochloride on influenza virus." XP002307894 retrieved from STN Database accession no. 1971:108598 & A. DENYS: "Effect of guanidine.HCl on influenza virus. I. Inactivation of influenza virus and some other viruses" MEDYCYNA DOSWIADCALNA I MIKROBIOLOGIA, vol. 22, no. 3, 1970, pages 243-248,
- DATABASE WPI Week 200367, Derwent Publications Ltd., London, GB; AN 2003-701561 & JP 2003 180865 A (MITSUBISHI JUKOGYO KK) 02 July 2003
- COOLBEAR T ET AL: "LABORATORY-SCALE INVESTIGATIONS INTO THE USE OF EXTREMELY THERMOPHILIC PROTEINASES FOR CLEANING ULTRAFILTRATION MEMBRANES FOULED DURING WHEY PROCESSING", JOURNAL OF MEMBRANE SCIENCE, ELSEVIER SCIENTIFIC PUBL.COMPANY. AMSTERDAM, NL, vol. 67, no. 1, 4 March 1992 (1992-03-04), pages 93-101, XP000271953, ISSN: 0376-7388, DOI: 10.1016/0376-7388(92)87043-W

## Description

### BACKGROUND OF THE INVENTION

### 1. Field of the Invention

The present invention relates to a method for inactivating viruses and to a use of a virus inactivating agent in a filter and in an air conditioner unit equipped with the filter.

### 2. Description of the Related Art

Infectious diseases caused by viruses have been problems not solved yet and influenza epidemic, for example, becomes a hot topic in the mass media every year. To reduce the spread of influenza, pharmaceuticals effective therefore have been developed and there is a high possibility of preventing infection by inoculation of a vaccine prior to its spread. Recently, the SARS virus appeared suddenly and caused an uproar. Appearance of such an unknown virus leads to infection of a large number of people, because a vaccine to the virus cannot be developed so quickly. In addition, there is a fear of artificial infection of a virus such as smallpox which is thought to be eradicated already through bioterrorism or invasion of infection. If so, people not immune such as people unvaccinated with smallpox are in danger of death. We, human beings, can take countermeasures against such unknown viruses or viruses slipped from our memory, for example, by (1) avoiding going to areas in which we may be being infected with the virus, (2) avoiding contact with persons who may be infected, or the like. These measures are however not realistic, because they might reduce the productivity of society as a whole.

It is generally effective to spray alcohols, alkali compounds or hypochlorous acid for the inactivation of viruses. This method however brings about only temporary inactivation of viruses. In addition, chemicals such as acid and alkali are highly dangerous for organisms including human beings so that careful treatment is required.

If a constant virus-inactivating method is developed, it is possible to develop a highly efficient virus inactivating system which can be used for, for example, air conditioner units. Even if there is a danger of viruses spreading widely, a closed space having such an air conditioner equipment can be maintained safely. International Publication WO 98/04334 discloses an air cleaning filter having an enzyme fixed thereon. The air cleaning filter is effective for killing microorganisms but is poor in virus removing capacity, because it uses only an enzyme.

### SUMMARY OF THE INVENTION

With the foregoing in view, the present invention has, as an object thereof, to provide a virus inactivating method, which can be applied to an air conditioner and can inactivate a viruse effectively and continuously. Another object of the present invention is to provide the use of a virus inactivating agent in a filter set having the inactivating agent supported thereon and an air conditioner equipped with the filter.

In order to attain the above-described objects, the present invention provides, in one aspect
1. A method for inactivating a virus, comprising a step of exposing a virus to a solution containing a virus inactivating agent,
   wherein the virus inactivating agent is a virus inactivating agent for inactivating viruses in a liquid phase comprising as active ingredients a protein denaturant and the proteolytic enzyme pfu protease S.
2. The method according to item 1, wherein the protein denaturant is urea.
3. The method according to item 1, wherein the protein denaturant is a surfactant.
4. The method according to item 3, wherein the surfactant is sodium dodecyl sulfate (SDS).
5. The method according to any one of items 1 to 4, wherein the virus has an envelope.
6. The method according to any one of items 1 to 4, wherein the virus has not an envelope.
7. The method according to any one of items 1 to 6, wherein the virus inactivating agent has a bactericidal action.
8. The method according to any one of items 1 to 7, wherein the virus inactivating agent has a fungicidal action.
9. Use of a virus inactivating agent comprising as active ingredients a protein denaturant and the proteolytic enzyme pfu protease S for inactivating viruses in a liquid phase in a virus inactivating filter set, the virus inactivating filter set comprising a filter for trapping a virus and the virus inactivating agent adhered to the filter.
10. Use of a virus inactivating agent comprising as active ingredients a protein denaturant and the proteolytic enzyme pfu protease S for inactivating viruses in a liquid phase in an air conditioner unit, the air conditioner unit comprising: an air intake for introducing air therefrom; a heat exchanger for heating or cooling the air thus introduced from the air intake by heat exchange between the air and a refrigerant; an air outlet for releasing therefrom the air heat-exchanged by the heat exchanger; an air blower for promoting the release of the air from the air outlet; the virus inactivating filter set comprising a filter for trapping a virus and the said virus inactivating agent adhered to the filter, disposed in an inside space through which air flows; and a virus-inactivating-agent activating means for generating an atmosphere in the inside space suitable for the activation of the virus inactivating agent.
11. The use according to item 10, wherein the air conditioner unit further comprises a switching means for closing a part or the whole of an opening leading to the inside space so as to keep the inside space semi-closed or hermetically closed.
12. The use according to item 11, wherein the air suitable for the activation of the virus inactivating agent is agitated by the air blower in the hermetically closed space.
13. The use according to any one of items 10 to 12, wherein the virus-inactivating-agent activating means is for heating and evaporating water condensed in a cooling operation of the heat exchanger, in a heating operation of the heat exchanger conducted after the cooling operation.
14. The use according to any one of items 10 to 12, wherein the virus-inactivating-agent activating means is for heating and evaporating water condensed in a cooling operation of the heat exchanger and accumulated in a drain pan, by a heater.
15. A use according to item 13 or 14, wherein after the inside space is maintained hot and humid by the virus-inactivating-agent activating means, a deterioration preventive operation is conducted to remove water from the filter.
16. A use according to any one of items 10 to 15, wherein prior to the activation of the virus inactivating agent, a virus trapping operation is conducted by introducing air into the inside space, and causing the air to pass through the filter set.
17. A use according to any one of items 10 to 16, wherein the air conditioner unit further comprises an internal air retaining means for retaining the internal air in the inside space.

EP 1460348 A1 relates to an air-conditioning apparatus for deactivating antigens including an allergen deactivation filter containing an enzyme.
JP 2-041166 A relates to a filter containing lytic enzymes and to its use in air conditioners for the removal of pathogens such as bacteria.
JP 60-049795 A relates to synthetic fibres on which a bacteriolytic enzyme has been immobilized. The fibres of JP 60-049795 A can be used for air cleaning.
WO 94/00166 relates to a method for producing viral protective barrier materials containing surfactants for use in a clinical setting.
JP 2003-180 865 A discloses the use of a combination of protease enzymes, such as pfu protease S, and urea for inactivating allergens such as mites in filters.

### BRIEF DESCRIPTION OF THE DRAWINGS

FIG. 1 is a graph showing an inactivation ratio of λ phage.
FIG. 2 is a graph showing an inactivation ratio of M13 phage.
FIG. 3 is a graph showing a change in absorbance of an *Escherichia coli* suspension.
FIG. 4 is a graph showing a ratio of λ phage inactivated by an inactivating filter set.
FIG. 5 is a graph showing the infectious capacity of λ phage when the inactivating filter set was used.
FIG. 6 is a graph showing a ratio of M13 phage inactivated by an inactivating filter set.
FIG. 7 is a graph showing the infectious capacity of M13 phage when the inactivating filter set was used.
FIG. 8 illustrates the dry test method for fungi.
FIG. 9 illustrates the results of the dry test for fungi.
FIG. 10 illustrates the results of the wet test for fungi.
FIG. 11 is a cross-sectional view illustrating a first embodiment of an air conditioner indoor unit according to the present invention.
FIG. 12 is a perspective view illustrating an air conditioner according to the present invention.
FIG. 13 is a circuit diagram of a refrigerant flow in the air conditioner as illustrated in FIG. 12.
FIG. 14 illustrates a first example of a virus inactivating filter set, in which (A) is an overall view and (B) is a partially enlarged view of (A).
FIG. 15 illustrates a second example of the virus inactivating filter set and is a fragmentary view of the virus inactivating filter set.
FIG. 16 illustrates further examples of the virus inactivating filter set. (A) is an overall view illustrating a third constitution example of the virus inactivating filter set and (B) is an overall view illustrating a fourth example of the virus inactivating filter set.
FIG. 17 is a plan view illustrating the virus inactivating filter set, as illustrated in any one of FIGS. 14 to 16, housed in a case.
FIG. 18 illustrates further examples of the virus inactivating filter. (A) is an overall view illustrating a fifth example of the virus inactivating filter body, (B) is an overall view illustrating a sixth example of the virus inactivating filter set, and (C) is an overall view illustrating a seventh example of the virus inactivating filter set.
FIG. 19 is a plan view illustrating a specific example of a remote controller.
FIG. 20 is a cross-sectional view illustrating a modification example of the air conditioner indoor unit as illustrated in FIG. 11,
FIG. 21 is a fragmentary cross-sectional view illustrating a second embodiment of the air conditioner indoor unit according to the invention. and
FIG. 22 is a plan view illustrating the reservoir of FIG. 21.

### DETAILED DESCRIPTION OF THE PREFERRED EMBODIMENT

### 1. Virus inactivating agent

A virus is an infectious substance which generally comprises of protein and nucleic acid as basic components. The present invention is based on the finding by the present inventors that a protein denaturant and the proteolytic enzyme pfu protease S as active ingredients can inactivate a virus without ill effect on human bodies, with long lasting inactivation effects and excellent economy. The virus inactivating agent as used according to the present invention is suitable for practical application, because it can easily be incorporated in appliances such as an air conditioner.

The virus inactivating agent as used in the present invention has, in addition to a virucidal action, a microbicidal, especially bactericidal action and also fungicidal action against fungi such as molds.

The protein denaturant used in the present invention can denature the protein of a virus to inactivate the virus. The protein denaturant may include urea, guanidine hydrochloride, and surfactants such as sodium dodecyl sulfate (SDS).

The proteolytic enzyme generally refers to enzymes having properties of degrading proteins and peptides. The proteolytic enzyme to be used in the present invention can degrade the protein in a virus to inactivate the virus. The proteolytic enzyme is pfu protease S. Particularly preferred is pfu protease S sold by Takara Bio co. Ltd., which has high heat resistance and high durability in the presence of a denaturant such as urea or SDS.

When a virus is inactivated by the protease, conditions of the enzyme such as temperature or coenzyme may be optionally selected considering the optimum conditions of the protease.

In the present invention, the term "inactivation of a virus" refers to the deprivation of the infectious capacity from the virus. The virus can be deprived of its infectious capacity by denaturing or degrading the protein of the virus by using an active ingredient such as the above-described denaturant or enzyme. The term "bactericidal action" or "fungicidal action" refers to capacity of killing bacteria or fungi, or disturbing its growth.

The reaction by the enzyme or denaturant is preferably conducted in a liquid phase. The virus inactivating agent of the present invention is therefore preferably employed in a solution or in a liquid phase.

In another aspect, the present invention has been completed based on a unique new idea that the proteolytic enzyme and protein denaturant are used in combination for the virus inactivation. An important point in the aspect of the invention, therefore, resides not in specified conditions such as concentration of them or treating time with them, but in virus inactivation itself by combination of the proteolytic enzyme and protein denaturant. In other words, according to the present invention any condition such as types and concentrations of the proteolytic enzyme and of the protein denaturant is applicable.

Some viruses may have lytic enzymes having similar effects as that of the proteolytic enzyme. Such viruses have resistance to these enzymes as is obvious so that they cannot be inactivated even by the proteolytic enzyme of the present invention. In that case, however, use of the protein denaturant may be advantageous, because it facilitates the proteolytic enzyme to act on the virus resistant to the enzyme by denaturing the protein of the virus. In addition, use of the protein denaturant and proteolytic enzyme in combination enables a reduction in the concentration of the proteolytic enzyme.

As described above, in one aspect of the present invention, viruses can be inactivated more efficiently, as demonstrated later in Examples, by using both the protein denaturant and proteolytic enzyme.

Some viruses may have an envelope which is a membrane structure surrounding the outside of the protein capsid. The envelop is, similar to a cell membrane, a lipid bilayer membrane and has a virus-specific protein thereon. The present invention can be applied to both viruses with an envelope and those without an envelope, whereby an inactivating agent and inactivating method effective for a broad range of viruses can be provided.

### 2. Virus inactivating method

A description will next be made of a virus inactivating method using the protein denaturant and proteolytic enzyme.

When a virus is inactivated by the protein denaturant, urea can be used for example as the denaturant. Urea is preferably used at a concentration as high as about 9M.

When a virus is inactivated by the proteolytic enzyme, pfu rotease S is used. The concentration is preferably 2wt% in terms of final concentration when pfu protease S (Takara Bio co. Ltd., co. Ltd.,) is employed. The inactivation of viruses by the protease may depend on various conditions including temperature and time. The concentration of the protease is not limited to the above-described value but can be used effectively under various conditions.

The inactivation of a virus can be carried out efficiently in the presence of both the protein denaturant and proteolytic enzyme. When pfu protease S (Takara Bio co. Ltd.,) is used , a final concentration of about 2wt% may be preferable upon its single use. In the presence of 9M urea, however, effects sufficient for inactivating viruses can be obtained even at a concentration of about 0.2wt%.

### 3. Application to an air conditioner

The virus inactivating method of the present invention can be applied to an air conditioner. In this aspect, the present invention provides a virus inactivating filter set and an air conditioner having the filter set attached to an air conditioner indoor unit. Examples of the air conditioner using the filter set may include not only those for home use but also those for business use, for example, for buildings and those for vehicles.

### (1) Virus inactivating filter set

The virus inactivating filter set as disclosed herein comprises a filter for trapping viruses and a virus inactivating agent adhered to the filter. The filter for trapping viruses can be made, for example, of a nonwoven fabric. The virus inactivating agent contains as active ingredients a protein denaturant and the proteolytic enzyme pfu protease S. The virus inactivating agent adhered to the filter can inactivate viruses which are trapped by the filter. The virus inactivating agent can be adhered to the filter so that they are supported directly by the filter or supported by a carrier such as particles in a manner as described later.

The virus inactivating agent can inactivate viruses in a liquid phase. A hygroscopic material as a carrier such as filter fibers or particles can generate a fine liquid phase on the surface of the fibers or inside of the carrier particles. Thus, the inactivating agent can be activated.

The material for the filter may include fibers of, for example, polyethylene (PE), polypropylene (PP), polyethylene terephthalate (PET) and polyamide (PA) . The material for the carrier may include water absorbing polymers such as acrylic acid-vinyl alcohol copolymers and acrylic acid-sodium polymers.

When the filter is of hygroscopic fiber, they may be used together with water repellent fiber. The water repellent fiber can be effective for maintaining the shape of the filter even though the filter absorbs much humidity. The filter can be provided in various forms such as pleated form. The water repellent fiber may be particularly effective when the filter has a pleated form.

Even when the filter becomes wet by immersion in or spraying of the inactivating agent, the shape of the filter containing the water repellent fiber is not damaged. It is therefore important to apply the water repellent fiber in combination with the hygroscopic fiber to the filter before the inactivating agent is adhered to the filter.

Since the shape of the filter can be maintained without damage, the supported amount of the inactivating agent per unit area of the filter can be made uniform.

Although the filter set installed to an air conditioner may be exposed to the external force by wind pressure, the water repellent fiber can increase the strength of the filter and thereby prevent the filter from changing its shape.

### (2) Air conditioner

The air conditioner according to the present disclosure comprises the above-described virus inactivating filter set. The virus inactivating filter set may be attached to an air conditioner indoor unit so that the air suctioned during the operation of the air conditioner may pass through the filter set. It enables trapping, in the filter set, viruses in the room to inactivate them.

### (Examples)

The present invention will hereinafter be described in further detail referring to Examples. In these Examples, fundamental experiments for confirming the effects of the virus inactivating agent were performed with viruses and bacteria which can be handled in a laboratory. An inactivation test of viruses, bacteria and fungi was conducted using a filter set comprising a filter and an inactivating agent supported thereon.

### 1. Inactivating agent and Inactivating Method for inactivation

### (1) Materials

As a typical example of a virus which can be handled in a laboratory, a virus having a bacterium as a host, that is, bacteriophage was employed. An experimental system using a bacteriophage and *Escherichia coli* is presumed to be a model of, for example, influenza virus and infection and proliferation of the virus in human beings. Inactivation of bacteriophage was evaluated by exposing the bacteriophage to an enzyme or denaturant in a liquid of a tube.

As the bacteriophage, λ phage and M13 phage were employed. These phages are most well-studied from the molecular biological viewpoint and they are frequently employed for cDNA library construction and gene expression analysis. The λ phage is a temperate phage. The phage can proliferate to kill a bacteria cell when it infects the cell (lytic infection), or can lysogenize the bacteria cell to be a prophage and can act together with the host cell (lysogenization). Thus, it is a phage having two life cycles. The M13 phage is a fibrous one-stranded DNA phage. It is released outside of the cell without causing lysis of the host cell after the proliferation therein. The host of λ phage may include XL1-Blue strain. The host of the M13 phage may include JM109 strain of *Escherichia coli (E. coli).* Both do not infect cell strains which do not have similar properties to those of the host. The test using phages with various properties or host can evaluate possibility for the application to a broad range of viruses. *Escherichia coli* was cultured on an LB medium (Table 1), followed by shaking culture overnight at 37°C in an Erlenmeyer flask.

**Table 1:**

| Composition of LB medium | |
|---|---|
| Yeast extract | 5 g |
| NaCl | 5 g |
| Trypton | 10 g |
| Water | 1000 mL |
| Agar (only solid medium) | 15 g |

### (2) Method

### <Example 1: Preparation of a λ phage solution>

By using λZAPII® vector kit (undigested) and Gigapack ® III Packaging kit (Toyobo co. Ltd.,), λ phage DNA was packaged to prepare λ phage in accordance with a manual attached to the kits.

*Escherichia coli (E. coli)* XL1-Blue MRF' was employed as a host strain. The XL1-Blue MRF' cultured in advance was infected with the λ phage, followed by further culturing. The culture medium was then centrifuged to obtain a stock solution of λ phage. A solution (10 µL) obtained by properly diluting the resulting λ phage stock solution was mixed with 100 µL of XL1-Blue MRF' (already washed with an SM buffer (Table 2) to OD less than 1) and the resulting mixture was incubated on the LB medium for 15 minutes at 37°C. To the resulting culture medium was added 4 mL of the LB medium containing 0.7wt% agarose ("Agarose L03", Takara Bio co. Ltd.,). The LB medium had been maintained at 45°C in a temperature controlled bath after autoclave sterilization and it will hereinafter be referred to as "top agar". After stirring, the resulting mixture was poured on the solid LB medium containing 1.5wt% agar which had been prepared in advance. After confirmation that the top agar was solidified by sufficient cooling, incubation was conducted overnight at 37°C.

After confirmation of the appearance of a plaque on the solid medium after incubation, 5 mL of an SM buffer was added and the mixture was allowed to stand for 1 day in a refrigerator. From the solid medium taken out from the refrigerator, the SM buffer was recovered and centrifuged (6000 × g, 20 minutes, 2°C). The supernatant thus obtained was provided for the subsequent inactivation test as a λ phage solution. A 0.5 mL portion of the λ phage solution was poured into a tube having a capacity of 1.5 mL (Eppendorf AG). The tube will hereinafter be referred to as "1.5-mL tube"). A drop of chloroform was added to the tube. The tube was stored at 4°C.

**Table 2:**

| SM buffer | |
|---|---|
| Tris-HCl (pH 7.5) | 50 mM |
| NaCl | 100 mM |
| MgSO₄ · 7H₂O | 10 mM |
| Gelatin | 0.01wt% |
| The SM buffer was sterilized in autoclave. | |

### <Example 2: Test on the Activity of λ phage>

A test on the activity of λ phage was performed and percent recovery was calculated in order to certify the establishment of the experimental system of λ phage to be used for the below-described tests.

To 40 µL of the λ phage solution prepared in Example 1 was added to a 360 µL of a phosphate buffered physiological saline (PBS) + 10 mM MgSO₄·7H₂O solution. PBS was used to suppress the change of pH, which might occur during the test. 10 mM MgSO₄ was added to minimize decrease in the infectious capacity of the λ phage, which is not caused by an inactivating agent.

The resulting solution was poured into a 1.5-mL tube. In the tube was poured 600 µL of a PEG solution (20wt/vol% PEG6000, 2.5M NaCl). The tube was allowed to stand for 3 hours in ice. The mixture was then centrifuged for 20 minutes at 15,000 rpm and the supernatant was discarded. To the precipitate thus obtained (λ phage·PEG composite) was added 200 µL of an SM buffer and the precipitate was suspended therein. The λ phage solution treated with the PEG is referred to as a PEG precipitated treating solution and the treatment with PEG is referred to as PEG treatment in the subsequent steps.

A solution (10 µL) obtained by diluting the PEG precipitated treating solution with an SM buffer was mixed with an SM suspension (100 µL) of XL1-Blue MRF' . The mixture was incubated at 37°C for 15 minutes. After completion of the incubation, 3 mL of the top agar was added and the mixture was stirred. The mixture was then poured on a solid LB medium containing 1.5wt% agar which had been prepared in advance. After incubation at 37°C for 5 hours, the number of plaques was counted. From the number, pfu (plaque forming unit, pfu/mL) was determined.

As a control, the XL1-Blue MRF' was infected with the λ phage solution prepared in Example 1 without PEG treatment and the number of the plaques was calculated to determine pfu. The results are shown in Table 3.

**Table 3:**

| Activity of λ phage | |
|---|---|
| Test conditions | Infectious capacity (pfu/mL) |
| PEG treatment | 1.9 × 10⁸ |
| No treatment | 9.2 × 10⁸ |

The PEG precipitated treating solution has a 1/5 concentration of the λ phage solution prepared in Example 1, because it was suspended in an SM buffer upon PEG treatment. Based on Table 3, the percent recovery of the λ phage after the PEG treatment was therefore (1.9×10⁸×5) / (9.2×10⁸) ×100 = 103%. The result suggests that the PEG treatment hardly disturbed the recovery of the λ phage. In addition, it has been confirmed that the stability of the λ phage was not impaired in the presence of PBS and MgSO₄·

### <Example 3: Inactivation Test 1 of λ phage>

Inactivation of the λ phage by a denaturant or an enzyme was tested. As the denaturant, urea was used. As the enzyme, pfu protease S (Takara Bio co. Ltd.,) was used.

To 40 µL of the λ phage solution prepared in Example 1 was added 352 mL of a PBS + 10 mM MgSO₄·7H₂O solution, followed by the further addition of 8 µL of pfu protease S (final concentration: 2wt%) . The resulting mixture was poured into a 1.5-mL tube. The mixture was used as a protease treated solution.

Separately, 360 µL of a PBS + 10 mM MgSO₄ · 7H₂O solution containing 9M urea was added to 40 µL of the λ phage solution. The resulting mixture was poured into a 1.5-mL tube and the mixture was used as a urea treated solution.

In addition, 352 µL of a PBS + 10 mM MgSO₄·7H₂O solution containing 9M urea was added to 40 µL of the λ phage solution, followed by the further addition of 8 µL of pfu protease S (final concentration 2wt%). The resulting mixture was poured into a 1.5-mL tube and the mixture was used as a urea·protease treated solution.

A mixture was obtained by adding 360 µL of PBS + 10 mM MgSO₄·7H₂O solution to 40 µL of the λ phage solution (control). The resulting mixture was used as an untreated solution.

Each of the resulting treated and untreated solutions was incubated at 37°C for 1 hour. After completion of the incubation, a 600 µL of a PEG solution (20wt/vol% PEG6000, 2.5M NaCl) was added. The mixture was allowed to stand for 3 hours in ice. The mixture was then centrifuged for 20 minutes at 15,000 rpm and the supernatant was discarded. To the precipitate thus obtained was added 200 µL of an SM buffer and the precipitate was suspended therein. A solution (10 µL) obtained by diluting the suspension with an SM buffer was mixed with an SM suspension (100 µL) of *Escherichia coli* XL1-Blue MRF'. The mixture was incubated at 37°C for 15 minutes. After completion of the incubation, 3 mL of the top agar was added. The resulting mixture was stirred and poured on a solid LB medium containing 1.5wt% agar which had been prepared in advance. After incubation at 37°C for 5 hours, the number of plaques appeared was counted and pfu was determined. The results are shown in Table 4.

**Table 4:**

| Infectious capacity of λ phage under various test conditions | |
|---|---|
| Test conditions | Infectious capacity (pfu/mL) |
| Treatment with protease | 1.8 × 10⁸ |
| Treatment with urea | 0 |
| Treatment with protease and urea | 0 |
| No treatment | 1.9 × 10⁸ |
| λ Phage solution | 9.2 × 10⁸ |

From the results, it has been found that λ phage was not inactivated by the protease treated solution, while it was inactivated by the urea treated solution containing 9M urea irrespective of the presence of protease. This suggests that the highly concentrated urea can inactivate the λ phage.

### <Example 4 (Reference): Inactivation test 2 of λ phage>

The inactivation test of the λ phage was carried out for various urea concentration and at various treating time.

The λ phage solution (40 µL) prepared in Example 1 was mixed with 352 µL of respective PBS + 10 mM MgSO₄·7H₂O solutions containing 0M, 3M and 9M urea. The resulting mixtures were poured into 1.5-mL tubes. Each of the mixtures and the λ phage solution (a control solution) was incubated at 37°C for 0, 15 and 60 minutes. After completion of the incubation, 600 uL of a PEG solution (20wt/vol% PEG6000, 2.5M NaCl) was added to the mixture and the mixture was allowed to stand for 3 hours in ice. The mixture was then centrifuged for 20 minutes at 15,000 rpm and the supernatant was discarded. To the precipitate thus obtained was added 200 µL of an SM buffer and the precipitate was suspended therein. A solution (10 µL) obtained by diluting the suspension with an SM buffer was mixed with an SM suspension (100 µL) of *Escherichia coli* XL1-Blue MRF'. The mixture was incubated at 37°C for 15 minutes. After completion of the incubation, 3 mL of the top agar was added to the mixture and stirred. The mixture was poured on a solid LB medium containing 1.5wt% agar which had been prepared in advance. After incubation at 37°C for 5 hours, the number of plaques appeared was counted and pfu was determined. The results are shown in Table 5.

**Table 5:**

| Infectious capacity of λ phage under various test conditions (pfu/mL) | | | | |
|---|---|---|---|---|
| Treating time (min) | Test conditions | | | |
| | 9M Urea | 3M Urea | 0M Urea | λ Phage solution |
| 0 | 4 × 10⁶ | 9.4 × 10⁷ | 1.1 × 10⁸ | 8× 10⁸ |
| 15 | 0 | 1.5 × 10⁸ | 1.1 × 10⁸ | |
| 60 | 0 | 1.1 × 10⁸ | 1.4 × 10⁸ | |

As described above, the λ phage solution contained λ phage 5 times as much as that of the other treatment solution. In the solution containing 0M urea, plaques about one fifth of the λ phage solution was observed. This suggests that the λ phage was not inactivated at all. It has also been confirmed that in the solution containing 3M urea, the λ phage was scarcely inactivated irrespective of the treating time. In the solution containing 9M urea, on the other hand, the λ phage was completely inactivated for 15 minutes or greater. It has been confirmed that even just after the addition of urea, the λ phage was inactivated to some extent. This suggests that the addition of 9M urea to the λ phage solution causes rapid inactivation of λ phage.

### <Example 5 (Reference): Inactivation test 3 of λ phage>

Based on the test results in Example 4 suggesting that highly-concentrated urea is effective for inactivation of λ phage, the inactivation test of λ phage was carried out for various treating time with 9M urea.

The λ phage solution (40 µL) prepared in Example 1 was mixed with 360 µL of a PBS + 10 mM MgSO₄·7H₂O solution containing 9M urea. The resulting mixture was poured into a 1.5-mL tube and incubated at 37°C for each of 0, 7.5, 15, 30 and 60 minutes. After completion of the incubation, 600 µL of a PEG solution (20wt/vol% PEG6000, 2.5M NaCl) was added and the mixture was allowed to stand for 3 hours in ice. The mixture was then centrifuged for 20 minutes at 15,000 rpm and the supernatant was discarded.

To the precipitate thus obtained was added 200 µL of an SM buffer and the precipitate was suspended therein. A solution (10 µL) obtained by diluting the suspension with an SM buffer was mixed with an SM suspension (100 µL) of *Escherichia coli* XL1-Blue MRF' and the mixture was incubated at 37°C for 15 minutes. After completion of the incubation, 3 mL of the top agar was added. The resulting mixture was stirred and poured on a solid LB medium containing 1.5wt% agar which had been prepared in advance. After incubation at 37°C for 5 hours, the number of plaques appeared was counted and pfu was determined. As a control, pfu of the λ solution free of the above-described treatment was determined in a similar manner. The results are shown in Table 6.

**Table 6:**

| Infectious capacity of λ phage when 9M urea was added | |
|---|---|
| Treating time (min) | Infectious capacity (pfu/mL) |
| 0 | 2.8 × 10⁵ |
| 7.5 | 0 |
| 15 | 0 |
| 30 | 0 |
| 60 | 0 |
| λ phage solution | 8.4 × 10⁸ |

It has been confirmed that most of the λ phage was inactivated just after exposed to 9M urea. The inactivation ratio reached about 99.96%. Since the λ phage was collected just after the addition of urea, the existence of 9M urea was presumed to have no influence on the percent recovery of the λ phage.

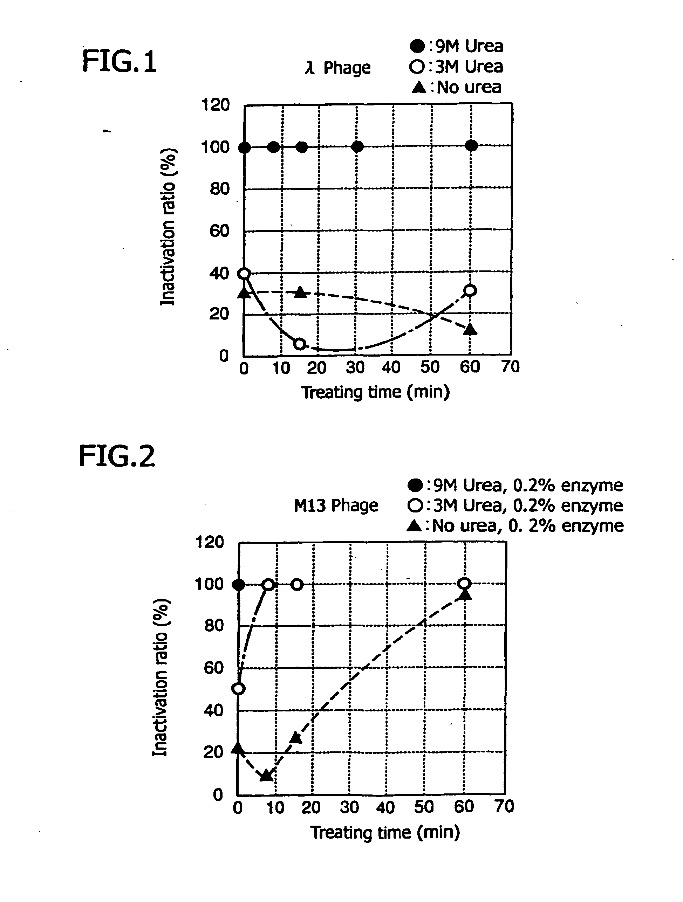

Based on the results of Tables 5 and 6, the inactivation ratio of the λ phage was calculated in accordance with the following formula: inactivation ratio = {1 - pfu of each treated solution/(pfu/5 of untreated solution)} x 100 and plotted on a graph of FIG. 1.

### <Example 6: Preparation of M13 phage solution>

M13p 18RFI (Toyobo co. Ltd.,) was used as the DNA of M13 phage. *Escherichia coli* JM109 was used as a host strain. The M13 phage DNA solution (1 µL) was mixed with 9 µL of sterilized distilled water, followed by transformation into competent cells of JM109 ("*E. coli* JM109, Toyobo co. Ltd.,) in accordance with the manual attached thereto. The *Escherichia coli* thus transformed was inoculated on an LB medium (10 mL) prepared separately and incubated overnight at 37°C under gentle shaking.

The culture medium was centrifuged (6000 g × 20 minutes, 4°C) to obtain a supernatant. The supernatant containing M13 phage was used as an MJ13 phage stock solution. The stock solution (10 µL) was added to 100 µL of an SM suspension (washed with SM to OD = ∼1) of JM109 which had been cultured separately, followed by incubation at 37°C for 15 minutes. To the resulting culture medium was added 4 mL of the top agar. After stirring, the resulting mixture was poured onto a solid LB medium containing 1.5wt% agar which had been prepared in advance. After confirmation that the top agar was solidified by sufficient cooling, incubation was conducted overnight at 37°C.

After the appearance of plaques on the solid medium was confirmed after incubation, 5 mL of an SM buffer was added and the mixture was allowed to stand for 1 day in a refrigerator. The solid medium was taken out from the refrigerator and the SM buffer was recovered and centrifuged (6000 g × 20 minutes, 2°C). The supernatant thus obtained a M13 phage solution was provided for the subsequent inactivation test. A 0.5 mL of the M13 phage solution was poured into a 1.5-mL tube. After the addition of a drop of chloroform, the mixture was stored at 4°C.

### <Example 7: Test on the activity of M13 phage>

A test on the activity of M13 phage was performed and a percent recovery was calculated in order to certify the establishment of the experimental system of M13 phage used for the below-described Examples.

To 40 µL of the M13 phage solution prepared in Example 6 was added 360 µL of a PBS + 10 mM MgSO₄·7H₂O solution. PBS was used to suppress change of pH, which might occur during the test. A 10 mM MgSO₄ was added to minimize decrease in the infectious capacity of the M13 phage, which is not caused by the inactivating agent.

The resulting solution was poured into a 1.5-mL tube. In the tube was poured 600 µL of a PEG solution (20wt/vol% PEG6000, 2.5M NaCl) and the tube was allowed to stand for 3 hours in ice. The mixture was then centrifuged for 20 minutes at 15,000 rpm and the supernatant was discarded. To the precipitate thus obtained was added 200 µL of an SM buffer and the precipitate was suspended therein. The M13 phage solution treated with the PEG solution was used as a PEG precipitated treating solution in the subsequent steps.

A solution (10 µL) obtained by diluting the PEG precipitated treating solution with an SM buffer was mixed with an SM suspension (100 µL) of JM109 and the mixture was incubated at 37°C for 15 minutes. After completion of the incubation, 3 mL of the top agar was added. The resulting mixture was stirred and poured on a solid LB medium containing 1.5wt% agar which had been prepared in advance. After incubation at 37°C for 5 hours, the number of plaques appeared was counted. From the number of plaques thus obtained, pfu (plaque forming unit, pfu/mL) was determined.

As a control, the JM109 was infected with the M13 phage solution prepared in Example 6 without PEG treatment and the number of the plaques was calculated to determine pfu. The results are shown in Table 7.

**Table 7:**

| Activity of M13 phage | |
|---|---|
| Test conditions | Infectious capacity (pfu/mL) |
| PEG treatment | 2.0 × 10¹⁰ |
| No treatment | 1.1 × 10¹¹ |

The PEG precipitated treating solution has a 1/5 concentration of the M13 phage solution prepared in Example 6, because it was suspended in an SM buffer in PEG treatment. Based on Table 7, the percent recovery of the M13 phage after PEG treatment was 91%. The result suggests that PEG treatment hardly disturbed the recovery of the M13 phage. In addition, it has been confirmed that the stability of the M13 phage was not impaired even in the presence of PBS and MgSO₄.

### <Example 8: Inactivation Test 1 of M13 phage>

Inactivation of the M13 phage by a denaturant or an enzyme was tested. As the denaturant, urea was used, while as the enzyme, pfu protease S (Takara Bio co. Ltd.,) was used.

To 40 µL of the M13 phage solution prepared in Example 6 was added 352 µL of a PBS + 10 mM MgSO₄·7H₂O solution, followed by the further addition of 8 µL of pfu protease S (final concentration of 2wt%). The resulting mixture was poured into a 1.5-mL tube and was used as a protease treated solution.

Separately, 360 µL of a PBS + 10 mM MgSO₄·7H₂O solution containing 9M urea was added to 40 µL of the M13 phage solution. The resulting mixture was poured into a 1.5-mL tube and was used as a urea treated solution.

In addition, 352 µL of a PBS + 10 mM MgSO₄ · 7H₂O solution containing 9M urea was added to 40 µL of the M13 phage solution, followed by the further addition of 8 µL of pfu protease S (final concentration of 2wt%). The resulting mixture was poured into a 1.5-mL tube and was used as a urea·protease treated solution.

As a control, a mixture was obtained by adding 360 µL of a PBS + 10 mM MgSO₄·7H₂O solution to 40 µL of the M13 phage solution prepared in Example 6. The resulting mixture was used as an untreated solution.

Each of the treated solutions and untreated solution was incubated at 37°C for 1 hour. After completion of the incubation, 600 µL of a PEG solution (20wt/vol% PEG6000, 2.5M NaCl) was added to the mixture. The mixture was allowed to stand for 3 hours in ice. The mixture was then centrifuged for 20 minutes at 15,000 rpm and the supernatant was discarded. To the precipitate thus obtained was added 200 µL of an SM buffer and the precipitate was suspended therein. A solution (10 µL) obtained by diluting the suspension with an SM buffer was mixed with an SM suspension (100 µL) of *Escherichia coli* JM109. The mixture was incubated at 37°C for 15 minutes. After completion of the incubation, 3 mL of the top agar was added. The resulting mixture was stirred and poured on a solid LB medium containing 1.5wt% agar which had been prepared in advance. After incubation at 37°C for 5 hours, the number of plaques appeared was counted and pfu was determined. The results are shown in Table 8.

**Table 8:**

| Infectious capacity of M13 phage under various test conditions | |
|---|---|
| Test conditions | Infectious capacity (pfu/mL) |
| Treatment with protease | 0 |
| Treatment with urea | 1.1 × 10¹⁰ |
| Treatment with protease and urea | 0 |
| M13 phage solution | 1.1 × 10¹¹ |

From the results, it has been confirmed that M13 phage was inactivated completely by the protease having a final concentration of 2wt%; the inactivation ratio of M13 phage treated with 9M urea was about 50%: and M13 phage was inactivated completely in the system added with both protease and urea. This suggests that protease is effective for inactivation of M13 phage and combination of protease with urea is also effective. <Example 9: Inactivation test 2 of M13 phage>

The inactivation test of the M13 phage was carried out for various protease concentrations.

The M13 phage solution (40 µL) prepared in Example 6 was mixed with 352 µL of a PBS + 10 mM MgSO₄·7H₂O solution. To the resulting mixture poured into a 1.5-mL tube, pfu protease S (Takara Bio co. Ltd.,) was added in amounts of 0, 0.08, 0.8 and 8 µL, respectively. The final concentrations of the enzyme added to the solution were 0, 0.02, 0.2 and 2wt%, respectively.

The M13 phage solution (40 µL) prepared in Example 6 was mixed with 352 µL of a PBS + 10 mM MgSO₄·7H₂O solution containing 9M urea. To the resulting mixture poured into a 1.5-mL tube, pfu protease S (Takara Bio co. Ltd.,) was added in amounts of 0, 0.08, 0.8 and 8 µL, respectively. The final concentrations of the enzyme added to the solution were 0, 0.02, 0.2 and 2wt%, respectively.

Each treated solution was incubated at 37°C for 1 hour. After completion of the incubation, 600 µL of a PEG solution (20wt/vol% PEG6000, 2.5M NaCl) was added and the mixture was allowed to stand for 3 hours in ice. The mixture was then centrifuged for 20 minutes at 15,000 rpm and the supernatant was discarded. To the precipitate thus obtained was added 200 µL of an SM buffer and the precipitate was suspended therein. A solution (10 µL) obtained by diluting the suspension with an SM buffer was mixed with an SM suspension (100 µL) of *Escherichia coli* JM109. The mixture was incubated at 37°C for 15 minutes. After completion of the incubation, 3 mL of the top agar was added. The resulting mixture was stirred and poured on a solid LB medium containing 1.5wt% agar which had been prepared in advance. After incubation at 37°C for 5 hours, the number of plaques appeared was counted and pfu was determined. The results are shown in Table 9.

**Table 9:**

| Infectious capacity (pfu/mL) of M13 phage at various concentrations of urea and protease | | | |
|---|---|---|---|
| Concentration of protease (wt%) | Test conditions | | |
| | 0M Urea | 9M Urea | M13 phage solution |
| 0 | 1.0 × 10¹⁰ | 6.3 × 10⁹ | 2.2 × 10¹⁰ |
| 0.02 | 3.9 × 10⁹ | 0 | |
| 0.2 | 5 × 10⁸ | 0 | |
| 2 | 0 | 0 | |

With a rise in the concentration of protease, the number of plaques decreases. From the results, it was found that the inactivation of the M13 phage depends on the enzyme concentration. It has been revealed that the coexistence of 9M urea can enhance inactivation of M13 phage to obtain a high inactivation ratio even at a lower protease concentration.

### <Example 10: Inactivation test 3 of M13 phage>

The inactivation of M13 phage in the presence of both protease and urea was tested in further detail. Based on the results of Example 9, the inactivation of M13 phage was tested with 0.2wt% protease in various urea concentrations and various treating times.

To the M13 phage solution (40 µL) prepared in Example 6 was added 359 µL of PBS + 10 mM MgSO₄·7H₂O solutions containing 0M, 3M and 9M urea, respectively. To the resulting mixture poured into a 1.5-mL tube was added 0.8 µL (final concentration: 0.2wt%) of pfu protease S (Takara Bio co. Ltd.,).

Each treated solution was incubated at 37°C for 0, 7.5, 15, and 60 minutes, respectively. After completion of the incubation, 600 µL of a PEG solution (20wt/vol% PEG6000, 2.5M NaCl) was added to the mixture and the mixture was allowed to stand for 3 hours in ice. The mixture was then centrifuged for 20 minutes at 15,000 rpm and the supernatant was discarded. To the precipitate thus obtained was added 200 µL of an SM buffer and the precipitate was suspended therein. A solution (10 µL) obtained by diluting the suspension with an SM buffer was mixed with an SM suspension (100 µL) of *Escherichia coli* JM109 and the mixture was incubated at 37°C for 15 minutes. After completion of the incubation, 3 mL of the top agar was added. The resulting mixture was stirred and poured on a solid LB medium containing 1.5wt% agar which had been prepared in advance. After incubation at 37°C for 5 hours, the number of plaques appeared was counted, pfu was determined and infectious capacity was rated. The results are shown in Table 10.

**Table 10:**

| Infectious capacity (pfu/mL) of M13 phage in various urea concentrations with 0.2wt% protease | | | | |
|---|---|---|---|---|
| Treating time (min) | Test conditions | | | |
| | 9M Urea | 3M Urea | 0M Urea | M13 phage solution |
| 0 | 1 × 10⁸ | 1.1 × 10¹⁰ | 1.7 × 10¹⁰ | 1.1 × 10¹¹ |
| 7.5 | 0 | 0 | 2.0 × 10¹⁰ | |
| 15 | 0 | 0 | 1.6 × 10¹⁰ | |
| 60 | 0 | 0 | 1.1 × 10⁹ | |

It has been confirmed that in the presence of both protease and 9M urea, most of the M13 phage was inactivated just after exposure to them. In the presence of 3M urea, the inactivation ratio was only about 50% when the M13 phage was brought into contact therewith the active ingredients. However, the ratio increased to almost 100% after treating for 7.5 minutes. Without urea, the M13 phage was hardly inactivated even after 15 minutes of the addition of protease and it took about 1 hour to attain the inactivation ratio of 90% or greater.

Based on the results of Table 10, the inactivation ratio of the M13 phage was calculated in accordance with the following formula: inactivation ratio = {1 - pfu of each treated solution/(pfu/5 of untreated solution)} × 100 and plotted on a graph of FIG. 2.

It has been found by the above-described examples that the virus inactivating agent of the present invention is effective for inactivation of viruses. The protein denaturant and the proteolytic enzyme are also effective for inactivating viruses when used singly, but use of them in combination heightens the virus inactivating effect. Moreover, such a virus inactivating agent containing both is effective for various viruses.

### <Example 11: Sterilization Test 1 of Escherichia coli)

Effects of the inactivating agent were tested on sterilization of *Escherichia coli* JM109 (which will hereinafter be referred to as "*E*. *coli"*).

*E. coli* was incubated on an LB medium while shaking at 37°C for 10 hours. After confirmation that it was in a logarithmic phase of growth, it was recovered by centrifugal separation (5,000 g × 20 minutes, 37°C). The *E*. *coli* thus precipitated was suspended in PBS and recovered again by centrifugal separation (5,000 g × 20 minutes, 37°C) . The *E*. *coli* thus recovered was suspended in PBS to prepare an *E. coli* suspension of OD 600 = -15.

The resulting E. coli suspension (32 µL) was mixed with 8 µL of a pfu protease S (Takara Bio co. Ltd.,) solution (final concentration; 2wt%) and 160 µL of a PBS solution of 10M urea. In addition, a mixture of 32 µL of the *E*. *coli* suspension with 8 µL of a pfu protease S solution and 160 µL of a PBS solution; and a mixture of 32 µL of the *E. coli* suspension with 8 µL of a PBS solution and 160 µL of a PBS solution of 10M urea were prepared. As a control, a mixture of 32 µL of the *E. coli* suspension with 168 µL of a PBS solution was prepared. Each solution was poured to a 1.5-mL tube and incubated at 37°C for 1 hour.

After the incubation, each tube was centrifuged (10,000 g × 20 min) and the supernatant was discarded. The precipitate containing cells was suspended in 400 µL of a PBS solution. The resulting suspension was seeded on an LB solid medium to incubate *E. coli.* The number of colonies formed by the incubation at 37°C for 12 hours was counted. The count was compared with those obtained from *E. coli* not subjected to the above-described treatment and a lethality of *E. coli* was calculated.

**Table 11:**

| Lethality of *E. coli* under each test condition | |
|---|---|
| Inactivating ingredient | Lethality (%) |
| Protease + urea | 100 |
| Protease | 2.3 |
| None | 1.2 |

The lethality of *E*. *coli* treated only with a PBS solution as a control was a little greater than 1%, suggesting that the treatment in the Example hardly affected *E. coli.* In the Example, the lethality of *E. coli* was very small when treated only with pfu protease S, while it E. coli was dead completely when treated with 10M urea and protease used in combination.

### <Example 12: Sterilization Test 2 of E. coli>

Effects of the inactivating agent were tested on sterilization of *E. coli* by measuring absorbance of its suspension.

As in Example 11, a suspension of *E*. *coli* of OD 600 = ∼ 15 was prepared. The resulting suspension (160 µL) of *E.* coli was mixed with 40 µL of a pfu protease solution and 800 µL of a 10M urea PBS solution. The resulting mixture was poured into a 1.5-µL tube.

Separately, 160 µL of the *E. coli* suspension was mixed with 40 µL of a pfu protease solution and 800 µL of a PBS solution and the mixture was poured into a 1.5-µL tube similarly. As a control, 160 µL of the E. coli suspension was mixed with 840 µL of a PBS solution and the mixture was poured into a 1.5-µL tube. These tubes were each incubated at 37°C.

After incubation for 0, 5, 35 and 60 minutes, sampling was conducted from each tube, followed by dilution to 1:20 with PBS. The turbidity at 600 nm was measured using a spectrophotometer.

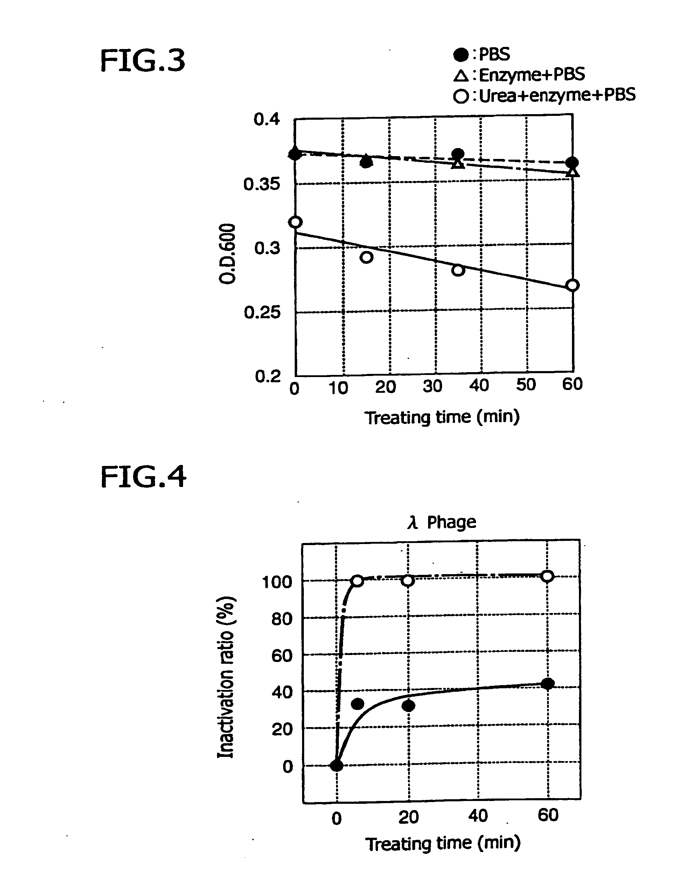

The results are shown in FIG. 3. An absorbance decrease means a decrease in an amount of bacteria existing in the diluted solution. The absorbance of the control solution showed almost no change throughout the test. The absorbance of the solution treated only with protease showed a slight decrease, while that of the solution treated with both protease and 10M urea showed a drastic decrease. These results suggest that the inactivating agent containing protease and urea was highly effective for sterilization of *E. coli.*

### 2. Examples of filter set and air conditioner

### (1) Inactivating filter set

Examples using the virus inactivating filter set according to the present invention will hereinafter be described.

### <Example 13: Process of producing virus inactivating filter set>

As a proteolytic enzyme, pfu protease S (Takara Bio co. Ltd.,) was used and an enzyme solution having a concentration of 2.1 mg/ml in terms of protein was prepared using PBS. A 10M aqueous urea solution was employed as a protein denaturant. The enzyme solution and urea solution were mixed, whereby a virus inactivating agent was prepared.

The virus inactivating agent attached to a filter in this Example is composed of 1.75 mL of the enzyme solution and 50 mL of the 10M urea solution, and the amount of the inactivating agent supported by the filter was 1.69. The amount of the inactivating agent supported by the filter was determined by the following formula: The amount of the inactivating agent supported = dry weight of the filter with an inactivating agent supported thereon/dry weight of the filter measured before applying an inactivating agent supported thereon.

The inactivating agent prepared as described above was adhered to "CELLFINE N" (Toyobo co. Ltd.,). After removing excess liquid, the filter was dried at normal temperature on a clean bench to adjust the supported amount to the above-described amount.

### <Example 14: Inactivation Test 1 of λ phage using inactivating filter set>

The inactivating filter set prepared in Example 13 was cut into a piece of 1.5 cm × 1.5 cm. The λ phage solution (40 µL) prepared in Example 1 was added dropwise to the filter set piece to infiltrate into the filter set uniformly. The filter set piece to which the phage solution was adhered was put into a sterilized 1.5-mL tube. The tube was covered with a lid, followed by incubation at 37°C. Incubation was conducted for 5 minutes, 20 minutes, or 60 minutes. After completion of the incubation, the 1.5-mL tube having the filter set therein was maintained in ice for 5 minutes.

An SM buffer (250 µL) which had been cooled in advance to 0 °C was poured into each 1.5-mL tube and the phage was recovered. In order to raise a percent recovery, 250 µL of an SM buffer was added again to recover the phage.

The total amount of the phage solution thus recovered was measured and 400 µL of it was transferred into a sterilized 1.5-mL tube. To this tube, 600 mL of a PEG solution was added, followed by incubation in ice for 1 hour.

After completion of the incubation, the tube was centrifuged for 20 minutes at 15000 rpm to remove the supernatant. To the tube in which the precipitate remained, 200 µL of an SM buffer was added to disperse the precipitate (composite of phage and PEG). By using the dispersed solution of the phage-PEG complex, the infectious capacity of the phage was rated in a similar manner to the inactivation test in the above-described examples.

As a control, an enzyme-immobilized filter "Biofree" (Nikki Universal co. Ltd.,) was subjected to a similar measurement. This filter "Biofree" is equipped with only an enzyme, but not protein denaturant.

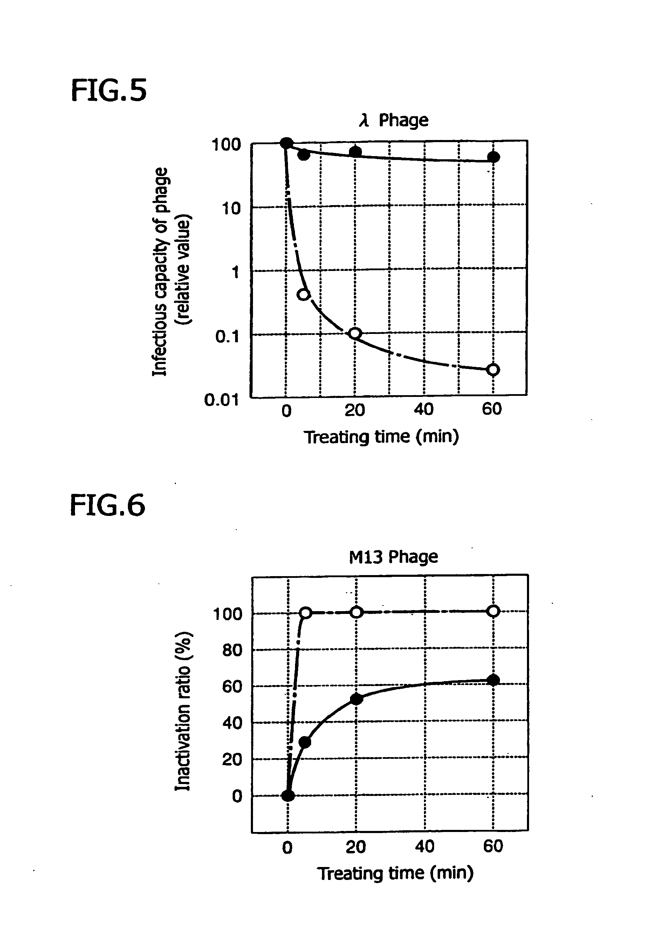

The results are shown in FIGS. 4 and 5. White circles (o) in these diagrams represent the measurement results using the filter set of the Example, black circles (•) represent the measurement results using a control filter. FIG. 3 illustrates an inactivation ratio of λ phage. In the diagram, an inactivation ratio without treatment was set at 0, while an inactivation ratio when the phage is completely inactivated was set at 100. The inactivation ratio was calculated in accordance with the following formula: inactivation ratio (%) = 100 - (infectious capacity × (total amount of the phage solution/40/2))/infectious capacity of λ phage solution), in which the term "infectious capacity" refers to the measurement result in the Example. FIG. 5 illustrates the infectious capacity where the infectious capacity of the original phage solution is taken as 100.

From the results thus obtained, it has been found that λ phage was almost completely inactivated in a short time by the inactivating filter set of the Example.

### <Example 15: Inactivation Test 2 of λ phage using inactivating filter set>

In Example 14, the phage attached to the filter was recovered in an SM buffer. In the case, if the phage remains on the filter, the phage recovered in the buffer decreases, which presumably leads to an apparent increase in the inactivation ratio of the phage. In the Example, therefore, an inactivation ratio of phage only by an inactivating agent was rated.

A filter set washed with water to remove the inactivating agent therefrom was provided for the test. In addition, the test was conducted at incubation temperature of 0°C, where the enzyme contained in the inactivating agent cannot work.

First, the inactivating filter set prepared in Example 13 was cut into a piece of 1.5 cm × 1.5 cm to use for the test under normal conditions. The λ phage solution (40 µL) was added dropwise to the filter set piece to infiltrate into the filter set. The filter set piece having the phage solution adhered thereto was put into a sterilized 1.5-mL tube. The tube was covered with a lid, followed by incubation at 37°C for 60 minutes. After completion of the incubation, the tube was maintained in ice for 5 minutes.

On the other hand, the inactivating filter set prepared in Example 13 was cut into a piece of 1.5 cm x 1.5 cm. The piece was washed with distilled water to remove therefrom the inactivating agent. The filter set thus washed was allowed to stand overnight at normal temperature and dried sufficiently. After drying, the phage solution was adhered to the washed filter set piece in a similar manner. The filter set was put into a sterilized 1.5-mL tube. The tube was covered with a lid, followed by incubation in ice for 60 minutes.

An SM buffer (250 µL) which had been cooled in advance to 0°C was poured into 1.5-mL tubes contained the filter set pieces exposed to different test conditions, respectively and the phage was collected from each tubes. In order to raise a percent recovery, 250 µL of an SM buffer was added again to recover the phage solution.

The total amount of the phage solution thus recovered was measured and 400 µL of it was transferred into a sterilized 1.5-mL tube. To the tube, 600 mL of a PEG solution was added, followed by incubation in ice for 1 hour.

After completion of the incubation, the tube was centrifuged for 20 minutes at 15000 rpm to remove the supernatant. To the tube in which the precipitate remained, 200 µL of an SM buffer was added to disperse the precipitate (complex of phage and PEG). By using the dispersed solution of the phage-PEG complex, the infectious capacity of the phage was rated in a similar manner to the inactivation test in the above-described Examples.

As a control, an enzyme-immobilized filter "Biofree" (Nikki Universal co. Ltd.,) was subjected to a similar measurement. This filter "Biofree" is equipped with only enzyme, but not protein denaturant.

The results are shown in Table 12.

**Table 12**

| Test conditions | 37 °C | 0°C, washed filter |
|---|---|---|
| Filter set of Example | 97.7% (± 2.7%) n=5 | 15.3% (± 9.7%) n=4 |
| Control filter | 42.1% (± 10.5%) n=3 | 32.4% (± 5.2%) n=3 |

In general, enzymes have almost no activity at 0°C. Accordingly, the inactivation ratio obtained at the incubation temperature of 0°C does not seem to be attributed to the activity of the enzyme but to be attributed to the phage remaining on the filter set without being recovered.

In the control filter, there was almost no difference between the inactivation ratio at 37°C and that at 0°C. This suggests that the inactivation ratio of the control filter is not attributed to the enzyme reaction.

On the other hand, when the filter set of the Example was used, a large difference in the inactivation ratio was observed between the incubation temperature at 0°C and that at 37°C. This suggests that the inactivation ratio is attributed to the inactivation of the phage by the inactivating agent.

### <Example 16: Inactivation test I of M13 phage using an inactivating filter set>

The test was performed in a similar manner to Example 14 except for the M13 phage solution prepared in Example 6 being used instead of the λ phage solution.

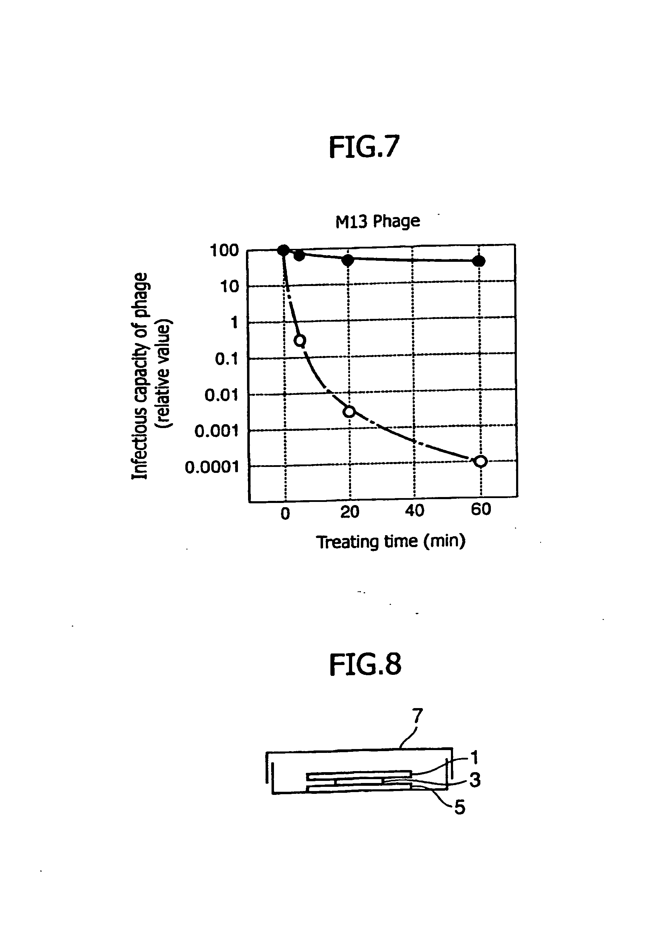

The results are shown in FIGS. 6 and 7. White circles (o) in the diagrams represent the measurement results when the filter set of the Example was used, while black circles (•) represent the measurement results when the control filter was used. FIG. 6 illustrates the inactivation ratio of M13 phage. FIG. 7 illustrates infectious capacity, where the infectious capacity of the original phage solution is taken as 100.

The results thus obtained have illustrated that M13 phage was inactivated almost completely in a short time by the inactivating filter set of the Example.

### <Example 17: Inactivation Test 2 of M13 phage by using an inactivating filter set>

A test was performed in a similar manner to Example 15, except for the M13 phage solution prepared in Example 6 being used instead of the λ phage solution.

The results are shown in Table 13.

**Table 13**

| Test conditions | 37°C | 0°C, washed filter |
|---|---|---|
| Filter set of the Example | 99.96% (± 0.14%) n=13 | 50.2% (± 16.5%) n=6 |
| Control filter | 62.969% (± 23.5%) n=11 | 57.4% (± 20.2%) n=4 |

In general, enzymes have almost no activity at 0°C. Accordingly, the inactivation ratio at 0°C does not seem to be attributed to the activity of the enzyme but to owe to the phage remaining on the filter without being recovered.

In the control filter, there was almost no difference between the inactivation ratio at 37°C and that at 0°C. This suggests that the inactivation ratio of the control filter is not attributed to the enzyme reaction.

On the other hand, a large difference in the inactivation ratio was observed between the incubation temperature at 0°C and that at 37°C. This suggests that the inactivation ratio attained by the filter set of the Example was attributed to the inactivation of the virus by the inactivating agent.

### <Example 18: Inactivation test of influenza virus by using an inactivating filter set>

Inactivation test of an influenza virus having an envelope membrane was performed using an inactivating filter set.

The inactivating filter set prepared in Example 13 was cut into a piece of 10 mm x 10 mm. Influenza virus type A/H1N1 (ATCC No. VR-95) was employed.

The influenza virus was inoculated in the allantoic cavity of an embryonated chicken egg and cultured for 7 days in an incubator of 37°C. The chorioallantoic fluid was collected and centrifuged (3000 rpm, 30 minutes), and the supernatant was collected. The supernatant thus obtained was used as a virus stock solution.

The virus stock solution was adhered to the inactivating filter set by using an atomizer. The amount of the virus stock solution applied to the filter set was 20 µL as calculated from a weight difference before and after adhesion of the virus solution. As a control, the virus stock solution was adhered in a similar manner to a filter having no inactivating agent supported thereon.

Each filter was placed in a desiccator having a relative humidity of 90%, followed by incubation at 35°C for 1 hour. After completion of the incubation, the filter was put into a sterilized 1.8-mL tube and 1 ml of sterilized PBS was added. The mixture was stirred for 5 minutes to extract the virus. The resulting virus extract solution was called "virus solution for evaluation".

MDCK cells derived from canine kidney were inoculated with the virus solution for evaluation, which was optionally diluted. Cytopathic effects of the MDCK cells (CPE, 5 days) were observed through an inverted microscope and TCID₅₀ (50% infectivity titer) was calculated. The TCID₅₀ of the test using an inactivating filter set was 1.77 ± 0.15 (n=3), while TCID₅₀ of the test using an untreated filter was 4.83 ± 0.06 (n=3).

### <Example 19: Inactivation test of polio virus using an inactivating filter set>

Inactivation test of a polio virus without an envelope membrane was performed using an inactivating filter set.

The inactivating filter set prepared in Example 13 was cut into a piece of 10 mm x 10 mm. Polio virus I Sabin strain (Lsc, 2ab) , an attenuated polio virus, was employed.

The green monkey kidney cells (BGM cells) were inoculated with polio virus and cultured for 4 days at 37°C. The culture medium was centrifuged (3000 rpm, 30 minutes), and the supernatant was collected. The supernatant thus obtained was used as a virus stock solution.

The virus stock solution was adhered to the inactivating filter set by using an atomizer. The amount of the virus stock solution adhered to the filter set was 20 µL as calculated from a weight difference before and after the adhesion of the virus solution. The virus stock solution was adhered to a filter having no inactivating agent supported thereon in a similar manner as a control.

Each filter was placed in a desiccator having a relative humidity of 90%, followed by incubation at 35°C for 1 hour. After completion of the incubation, the filter was put into a sterilized 1.8-mL tube and 1 ml of sterilized PBS was added. The mixture was stirred for 5 minutes to extract the virus. The resulting virus extract was called "virus solution for evaluation".

Green monkey kidney cells (BGM cells) were inoculated with the virus solution for evaluation, which was optionally diluted. Cytopathic effects of the BGM cells (CPE, 5 days) was observed through an inverted microscope and TCID₅₀ (50% infectivity titer) was calculated. The value of TCID₅₀ was 3.03 ± 0.15 (n=3) in the test using the inactivating filter set, while the value of TCID₅₀ was 5.03 ± 0.06 (n=3) in the test using the untreated filter.

### <Example 20: Sterilization test of E. coli using an inactivating filter set>

As in Example 12, a suspension of *E. coli* of OD600 = ∼ 15 was prepared. The resulting suspension (40 µL) was added dropwise to the inactivating filter set prepared in Example 13 uniformly. The suspension was added dropwise similarly to a filter not subjected to inactivation treatment as a control (untreated filter).

Each filter was put into a 1.5-mL tube and incubated at 37°C for 1 hour. After completion of the incubation, 200 µL of PBS was added to each tube, the mixture was centrifuged and *E. coli* bound to the filter was recovered. The operation was repeated twice.

The solution having *E. coli* recovered therein was centrifuged (10,000 g × 20 min) and the supernatant was discarded. The precipitate containing the cells was suspended in 400 µL PBS. The resulting suspension was seeded on an LB solid medium to culture *E. coli.* The number of colonies formed in incubation at 37°C for 12 hours was counted.

The number of the colonies was compared with that obtained from the *E. coli* suspension before treatment and a lethality was calculated. As a result, the lethality of the *E*. *coli* suspension treated with the inactivating filter set was 99.1%, while that of the *E. coli* suspension treated with the untreated filter was 57.5%. Comparing the results obtained from the inactivating filter with those obtained from the untreated filter, the lethality was calculated to be 97.9%.

### <Example 21: Fungicidal test (dry test) by using an inactivating filter set>

The fungicidal effect of the inactivating filter set of the present invention was tested referring to JIS Standards "Methods for test of Fungus Resistance" (JIS Z 2911: 2000). *Aspergillus niger* was employed.

Five loopfuls of fungus spores were suspended in 10 mL of sterilized water containing 0.1wt% Tween 80. The resulting suspension was used as a spore suspension. A nonwoven cloth (1.4 mm thick) made of dry-heat sterilized glass fibers (φ 12 mm) was dipped in the spore suspension, followed by drying in a clean bench to prepare a spore carrier.

In a sterilized Petri dish, the spore carrier was placed on the inactivating filter set prepared in Example 13 and then, a glass plate (5 mm thick, 50 mm × 50 mm) was put on the Petri dish to cover it (FIG. 8). The incubation was conducted at constant temperature of 35°C and constant humidity of RH80%.

A control test was conducted in a similar manner by being used an untreated filter having no inactivating agent supported thereon, instead.

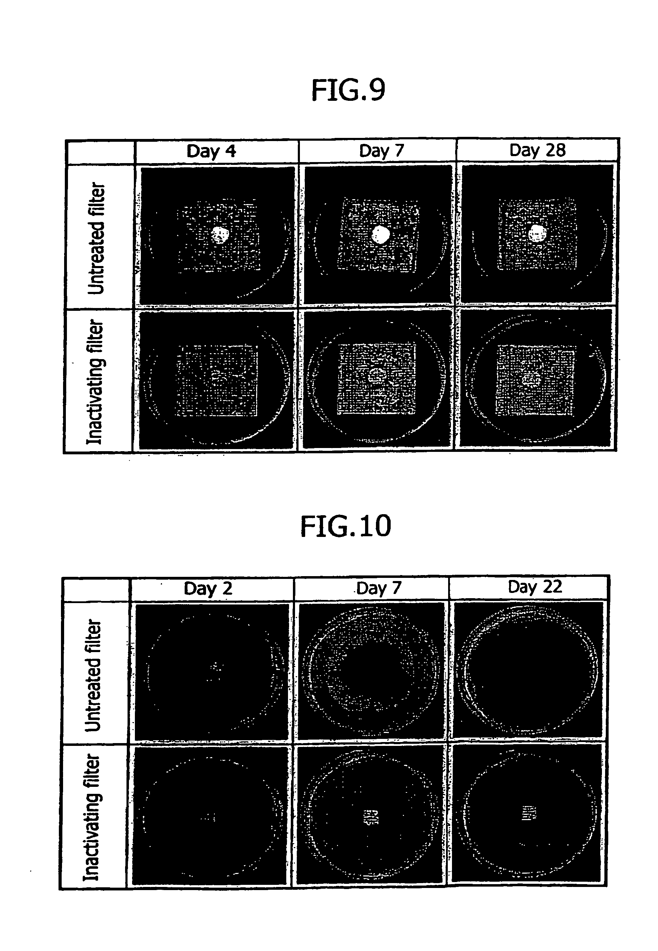

No growth of the fungi was observed both in the inactivating filter set and the untreated filter (FIG. 9).

Along with the above-described test, the spore suspension was cultured on a potato dextrose agar medium. As a result, growth of fungi was observed (data on it were not shown here). This suggests that the fungus spores themselves did not involve any problem in growth.

### <Example 22: Fungicidal test (wet test) using an inactivating filter set>

In a similar manner to that employed in Example 21, a spore suspension was prepared using *Aspergillus niger.*

The spore suspension (20 µL) was uniformly added dropwise to the inactivating filter set (10 mm × 10 mm) prepared in Example 13, followed by drying in a clean bench. Similarly, the spore suspension was added dropwise to the untreated filter, followed by drying. The resulting filter was used as a control.

Each filter to which fungus spores adhered was placed on a potato dextrose solid medium and incubated at a constant temperature of 35°C and constant humidity of RH 80%.

As a result of the observation, growth of the fungi was observed in the untreated filter, while no growth was observed in the inactivating filter set throughout the incubation period of 22 days (FIG. 10) .

Along with the above-described test, the spore suspension was cultured on a potato dextrose agar medium. As a result, growth of fungi was observed (data on it were not shown here) . This suggests that fungus spores themselves did not involve any problem in growth.

The above-described tests have revealed that the inactivating filter set of the present invention has fungicidal effects. The filter itself has not fungicidal capacity, because the growth of fungi was found in the untreated filter. The growth of fungi can be suppressed for the first time by the existence of the inactivating agent of the present invention. The above test also denies the possibility that the inactivating agent supported on the filter might serve as nutrient for fungus.

### (2) Air conditioner

An air conditioner indoor unit and air conditioner will hereinafter be described based on accompanying drawings.

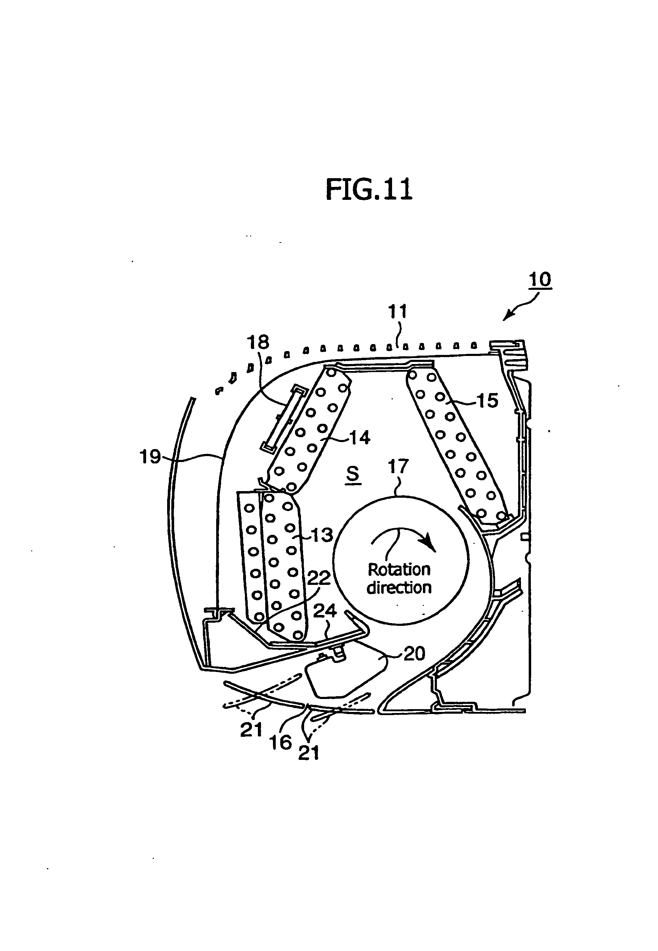

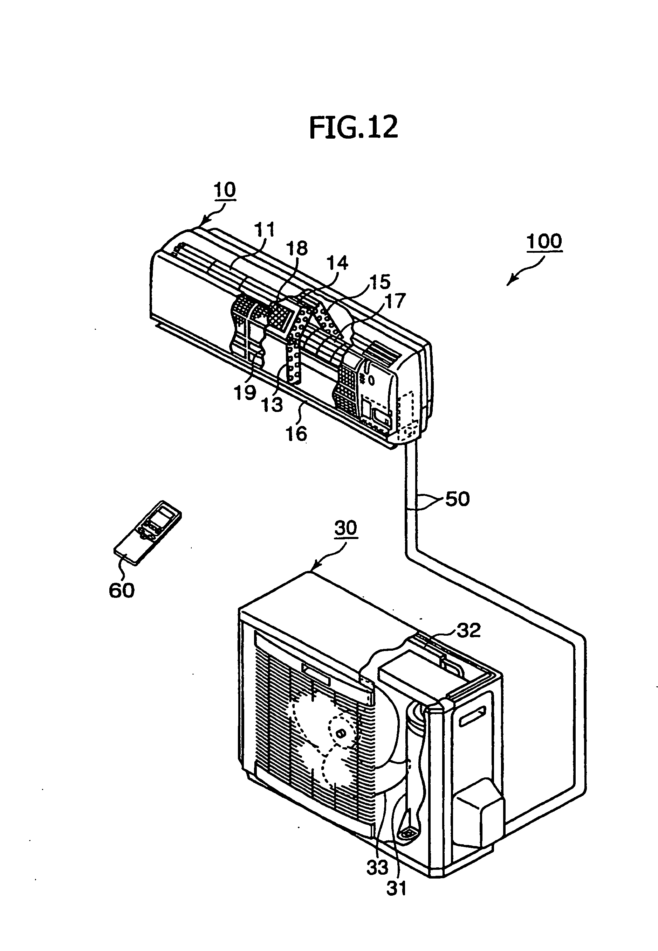

FIG. 11 is a cross-sectional view of an air conditioner indoor unit 10; and FIG. 12 is a perspective view illustrating the structure of an air conditioner 100 composed of the air conditioner indoor unit 10 and an air conditioner outdoor unit 30.

As illustrated in FIGS. 11 and 12, the air conditioner indoor unit 10 has, as main constituents, a suction grille (air intake) 11 for taking therein the air in the room, indoor heat exchangers 13, 14, and 15 for cooling or heating the air taken from the suction grille 11, an air outlet 16 for returning, to the room, the air which has been heat exchanged in the heat exchangers 13, 14 and 15, a cross flow fan (air blower) 17 for taking the air from the suction grille 11 and sending the heat-exchanged air to the room, and a virus inactivating filter set 18 disposed at a position in the upper vicinity of the upstream of the air passage in the indoor heat exchanger 14. A prefilter 19 is disposed from the front surface toward the upper surface of the inside of the air conditioner indoor unit 10. The prefilter can remove foreign matters such as dust from the air passing through the suction grille 11 and introduced into the heat exchangers 13, 14 and 15.

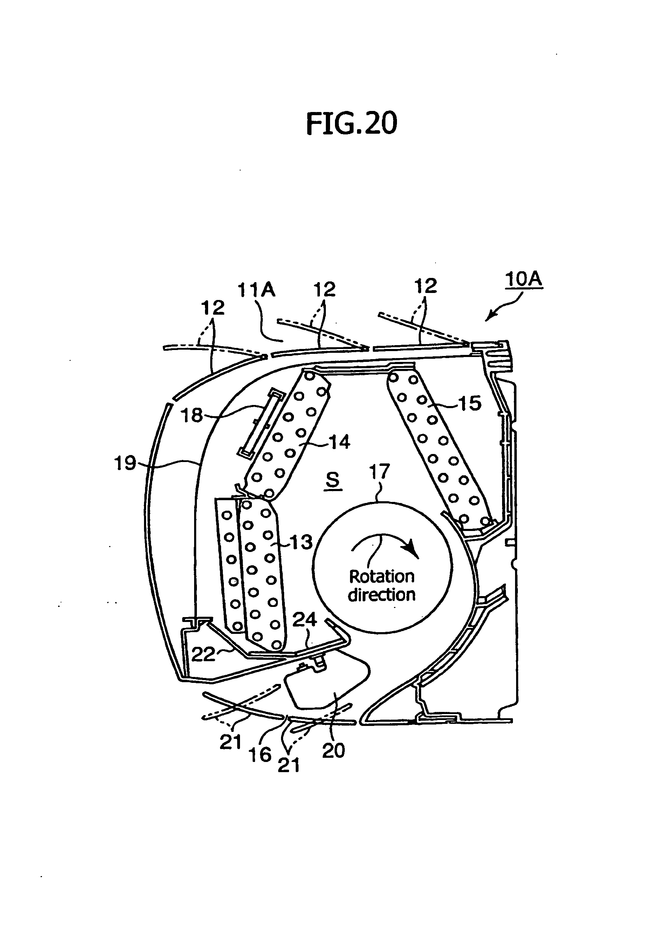

In the above-described air conditioner indoor unit 10, the suction grille 11, indoor heat exchangers 13, 14 and 15, air outlet 16, cross flow fan 17 and prefilter 19 are conventionally known. Thus, the description on them is omitted. The air outlet 16 is equipped with known louver 20 (shown in Fig. 20) and flap 21 for controlling the blowing direction. The air outlet 16 can be opened or closed by the movement of the flap 21.

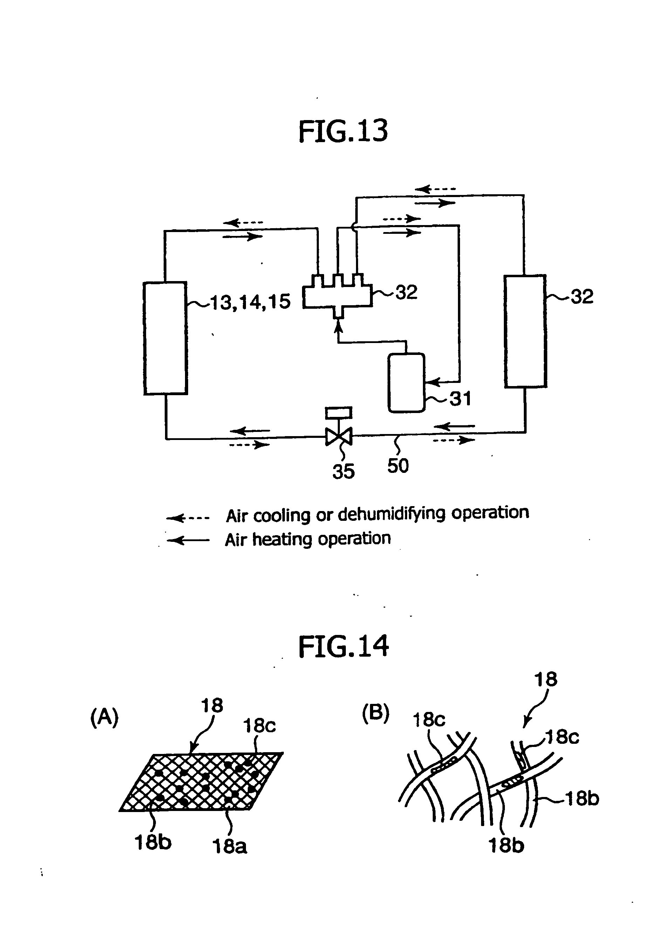

FIG. 12 is a schematic view illustrating the air conditioner 100 equipped with the above-described air conditioner indoor unit 10. In FIG. 12, the component indicated by symbol 30 is an air conditioner outdoor unit. The air conditioner outdoor unit 30 has a compressor 31 for compressing a refrigerant, an outdoor heat exchanger 32 for carrying out heat exchange between the refrigerant and outside air, and an outdoor fan 33 for promoting heat exchange in the outdoor heat exchanger between the refrigerant and outside air. A four-way valve 34 and electronic expansion valve 35 which will be described later referring to FIG. 13 are also disposed in the air conditioner outdoor unit 30.

Indicated at symbol 50 is a refrigerant pipe for connecting the air conditioner indoor unit 10 and the air conditioner outdoor unit 30 and circulating the refrigerant between the indoor unit 10 and the outdoor unit 30. Indicated at symbol 60 is a remote controller by which an operation mode of the air conditioner unit 100 can be set.

The virus inactivating filter set 18 has, for example, structures as shown in from FIG. 14 to FIG. 18.

FIG. 14 illustrates a first structural example of a virus inactivating filter set, in which FIG. 14(A) is an overall view, and FIG. 14(B) is a partially enlarged view of FIG. 14(A).

The virus inactivating filter set 18 can be equipped with a filter body 18a and a virus inactivating agent (which will hereinafter be called "inactivating agent" simply) 18c directly supported on fibers 18b making up the filter body 18a. Examples of the fibers 18b may include fibers such as glass, rayon, cellulose, polypropylene, polyethylene terephthalate, polyacrylic acid and polyacrylamide. Highly hygroscopic fiber, for example, CELLFINE N (Toyobo co. Ltd.,) can also be employed.

The inactivating agent 18c can be supported on the fibers 18b, not only in a physical process but also in a chemical process can be employed. For example, an active ingredient can be supported on a base material by converting the carboxyl group of the base material into azide and chemically binding the azide to the active ingredient via amide linkage. Not only the carboxyl group, but also a functional group such as hydroxyl or amino can be used for this chemical bonding. Such a chemically bearing method is conventionally known (Shin-jikken Kagaku Koza, Seibutsu Kagaku (1), pp. 363-409 Maruzen 1 (1978)).

By using the virus inactivating filter set 18 having the above-described structure, an amount of viruses to be inactivated can be increased drastically because the filter body 18a has, supported thereon, the inactivating agent 18c having an inactivating function of viruses.

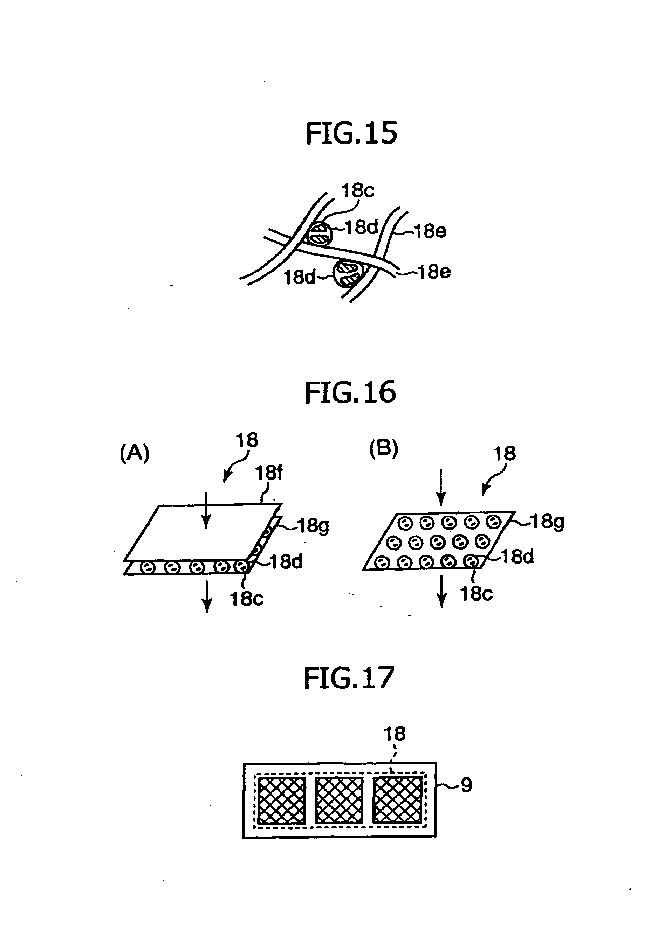

FIG. 15 is a fragmentary view of a second structural example of the virus inactivating filter set 18. The filter set illustrated in FIG. 15 comprises a carrier 18d having water absorption property and/or hygroscopic property with the inactivating agent 18c supported thereon, wherein the carrier 18d is fixed to fibers 18e with a binder (not illustrated). Examples of the material of the carrier 18d may include synthetic materials such as polyacrylic acid, polyacrylamide and polyvinyl alcohol; natural materials such as cotton, wool, sodium alginate, mannan and agar; and regenerated materials such as rayon. Examples of the material of the fibers 18e may include polymer compounds such as polyethylene (PE), polypropylene (PP), polyethylene terephthalate (PET) and polyamide (PA).

The virus inactivating filter set 18 of this Example has a structure in which the inactivating agent 18c is supported on the carrier 18d having water absorption and/or hygroscopic property and the carrier 18d is fixed to the fibers 18e using a binder so that the filter set can achieve similar effects to those of the above-described first structural example.

FIG. 16(A) illustrates a third structural example of the virus inactivating filter set 18, in which the inactivating filter set 18 is composed of a carrier 18d having a plurality of the inactivating agents 18c supported thereon and base materials 18f and 18g having the carrier sandwiched vertically therebetween.

Examples of the material of the carrier 18d may include polyacrylic acid, polyacrylamide, polyvinyl alcohol, cotton, wool, rayon, sodium alginate, mannan and agar. The base materials 18f and 18g may be each made of nonwoven fabric. The base material 18g which lies below the carrier 18d is preferably nonwoven fabric having a mesh smaller than the diameter of virus particles (particle size: several tens of microns to several hundreds of microns) .

The flat virus inactivating filter set 18 has a similar effect to that of the above-described structural examples, because the carrier 18d having the inactivating agent 18c supported thereon is sandwiched vertically between the two base materials 18f and 18g.

FIG. 16(B) illustrates a fourth structural example of the virus inactivating filter set 18. Although it is a flat virus inactivating filter set having a structure like open-sandwich as illustrated in FIG. 16(B), it can achieve a similar effect to that of the above-described examples.

The virus inactivating filter sets 18 having structures as illustrated in from the first to fourth structural example can be disposed, for example, in the air passage of the air conditioner indoor unit 10 contained in a case.

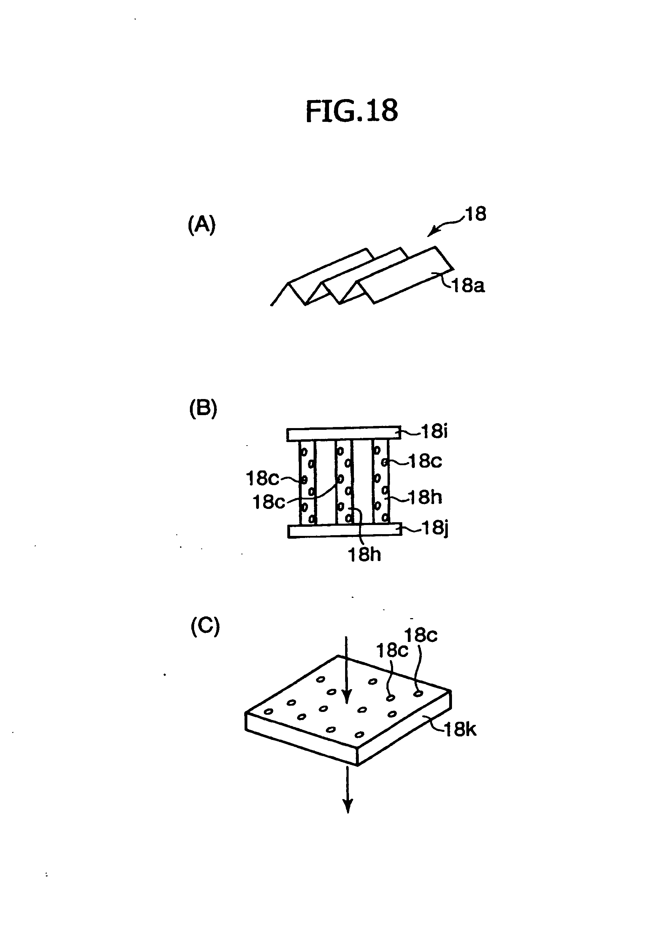

FIG. 18(A) illustrates a fifth structural example of the virus inactivating filter set 18. In this inactivating filter set 18, a filter body 18a is made of fibers having an inactivating agent directly supported thereon. The filter body 18a is in the pleated form.

According to the pleated virus inactivating filter 18, the filter body 18a is made of fibers having an inactivating agent directly supported thereon and the filter body 18a is in the pleated form so that the pressure loss is small compared with the filter sets of the above-described structural examples. The filter set has an improved virus trapping ratio because of an increase in the contact opportunities with viruses, and can suppress evaporation of water.

FIG. 18(B) illustrates a sixth structural example of the virus inactivating filter set 18. In the inactivating filter set 18, a plurality of rod members 18h with a circular cross-section are connected, at both ends thereof, to supporting members 18i and 18j, respectively. The rod members 18h is composed of bundled fiber having the virus inactivating agent 17c supported thereon.

In the rod type virus inactivating filter set 18 according to this structural example, the pressure loss is small. The filter set has a higher inactivating capacity owing to an increase in the supported amount of the inactivating agent, and has a longer operating life compared with the inactivating filter sets of from the first to fourth structural examples.

In this sixth structural example, each rod member has a circular cross-section. However, the rod member may have a triangular, square, elliptic or hollow cross-section. They may be arranged in a vertical direction, horizontal direction or oblique direction, or alternatively, they may be crossed each other. When the virus inactivating filter set 18 of this structural example is mounted on the air conditioner indoor unit 10, it is preferably attached to a position where air flows rapidly, for example, the air outlet 16 or both the suction grille 11 and air outlet 16.

FIG. 18(C) illustrates a seventh structural example of the virus inactivating filter set 18. The inactivating filter set 18 has the inactivating agent 18c supported on the surface of a porous body 18k such as urethane.

The virus inactivating filter set in the sponge form may have a similar effect to that of from the first to fourth structural examples.

The filter body may be made of water absorptive and/or hygroscopic materials are used. Examples include nonwoven fabric made of natural fibers such as cotton and wool, regenerated fibers such as rayon and cellulose acetate, or synthetic fibers such as polyethylene, polyethylene terephthalate and polyamide, cotton fabric, glass fiber mat, metal fiber mat, synthetic resins such as acrylic acid, acrylamide, and polyvinyl alcohol, and natural·regenerated materials such as sodium alginate, mannan and agar. The inactivating agent may be fixed to the filter body directly or via a carrier.

The virus inactivating agent used in the present invention can work in a liquid phase. It is possible to activate the inactivating agent by using, as the fibers or carrier of the filter, a water absorptive and/or hygroscopic material and thereby generating a fine liquid phase on the surface of the fibers or inside of the carrier.

In the above-described structures of the filter set, the inactivating agent may be active even at normal temperature and normal humidity, but the enzyme contained in the virus inactivating agent may be more active when the temperature is higher. Thus, the inactivating agent can be activated more under high-temperature and high-humidity atmosphere. The temperature is preferably within a range not exceeding the optimum temperature of the enzyme and at the same time, not greater than the heat resistant temperatures of the air conditioner and filter set. For the enzyme pfu protease S (Takara Bio co. Ltd.,), the temperature range is preferably from about 30 to 80°C.

The air conditioner indoor unit 10 equipped with the above-described virus inactivating filter set 18 may have an inactivating-agent activating means for maintaining the inside space S in a hot and humid atmosphere and thereby activating the inactivating agent. The term "inside space S" as used herein refers to a passage (space) of air sucked from the suction grille 11 and discharged from the air outlet 16. The inactivating-agent activating means according to a first embodiment, can operate the air conditioner 100 by effectively utilizing a component generally provided in an air conditioner without adding other components except for the virus inactivating filter set 18.

In the air conditioner 100, for example, a virus inactivating operation mode may be provided. In the mode, a refrigerant circuit provided with a conventional heat exchanger can work as an inactivating-agent activating means. A control means for the air conditioner 100 can carry out the operation mode to maintain the inside space S in a hot and humid atmosphere suitable for activation of the virus inactivating agent. The virus trapped by the virus inactivating filter set 18 may be destructed by the activated virus inactivating agent in advance, whereby the virus inactivation process for irreversible inactivation of the virus can be achieved.

In the virus inactivating operation mode, water is required for keeping hot and humid atmosphere. Cooling operation of the indoor heat exchangers 13, 14 and 15 disposed in the indoor air conditioner unit 10 is performed continuously for a predetermined time. Condensed water generated on the surface of these heat exchangers can be used for maintaining humid atmosphere. The cooling operation in the indoor heat exchangers 13, 14 and 15 can be carried out by circulating a refrigerant through a similar route to that upon cooling and dehumidifying operations for air conditioning where the heat exchangers can work as an evaporator. The cooling operation of the indoor heat exchangers will hereinafter be called "condensed water generating operation".

In the condensed water generating operation, as illustrated in the refrigerant circuit view of FIG. 13, a coolant can be circulated by driving a compressor 31 and outdoor fan 33 in the air conditioner outdoor unit 30. In the air conditioner indoor unit 10, the cross flow fan 17 is in operation while opening the flap 21 disposed in the air outlet 16.

The refrigerant is circulated in the following manner. As illustrated by the arrow (solid line) in FIG. 13, after the refrigerant is discharged from the compressor 31, the circulating direction is switched to a selected one by a four-way valve 34, and the refrigerant is circulated in a clockwise direction in the order of from the outdoor heat exchanger 32, electronic expansion valve 35, indoor heat exchangers 13, 14 and 15 and four-way valve 34 and finally returns to the compressor 31. The flow of the refrigerant can provide a gas-liquid two-phase flow with the indoor heat exchangers 13, 14 and 15. Heat exchange with air therefore is carried out so that air from which vaporization heat is removed becomes cool and moisture in the air condenses by a temperature decline to deposit on the surfaces of the heat exchangers as condensed water. The condensed water thus generated drips from the surfaces of the indoor heat exchangers 13, 14 and 15 to a drain pan 22. Then, the condensed water is discharged outside from the air conditioner indoor unit 10 through a drain passage which is not illustrated.

After completion of the condensed water generating operation, the operation mode is switched to heating operation (this heating operation will hereinafter be called "condensed water heating operation" in order to distinguish it from heating operation for air conditioning) in order to evaporate the condensed water thus generated and increase the temperature and humidity in the inside space S.

In the condensed water heating operation, as illustrated by the arrow (broken line) in the refrigerant circuit diagram of FIG. 13, the refrigerant discharged from the compressor 31 can flow in a direction opposite direction to that in condensed water generating operation, that is, counterclockwise direction by the switching operation of the four-way valve 34. The refrigerant discharged from the compressor 31 comes out of the four-way valve 34, then flows in the order of from the indoor heat exchangers 13, 14 and 15, electronic expansion valve 35, outdoor heat exchanger 32 and four-way valve 34, and returns to the compressor 31.

As described above, even in the condensed water heating operation, the high-temperature and high-pressure gas refrigerant supplied to the indoor heat exchangers 13, 14 and 15 can condense by heat exchange with air when the refrigerant is circulated similarly to an air heating operation (heating operation for air conditioning will hereinafter be called "air heating operation" to distinguish it from condensed water heating operation). As a result, the indoor heat exchangers can radiate heat as a condenser so that the condensed water deposited on the surfaces of the heat exchangers can be vaporized by utilizing the radiated amount of heat as a heating means.

In condensed water heating operation different from in air heating operation, the condenser 31 and outdoor fan 33 of the air conditioner outdoor unit 30 can be operated to promote vaporization of condensed water. However, the operation of the cross flow fan 17 may be stopped in the air conditioner indoor unit 10. At the same time, the flap 21 is operated to close the air outlet 16. As a result, the inside space S of the air conditioner indoor unit 10 is semi-closed with the air outlet 16 being closed, whereby the temperature in the inside space S is increased by the heat released from the indoor heat exchangers 13, 14 and 15. Further, the vapor of the condensed water generated by the heat released from the indoor heat exchangers 13, 14 and 15 stays in the inside space S to raise the humidity. This facilitates the formation of hot and humid atmosphere suitable for activating the virus inactivating agent(virus inactivating atmosphere).

In order to allow the virus inactivating filter set 18 to absorb humidity stably, the virus inactivating filter set 18 can be disposed above the indoor heat exchangers 13, 14 and 15. More preferably it is disposed just above the indoor heat exchangers to facilitate formation of a vapor passage, because vapor obtained by the vaporization of condensed water can go upward substantially directly.

The position of the inactivating filter set 18 is not always limited to the position above the indoor heat exchangers. It is disposed at least in an air flow route in the ordinary air-cooling or air-heating operation. At the same time, it is disposed at a position where the filter can contact with the evaporated air formed by the condensed water heating operation, inside of the indoor unit.

As the inside space S becomes atmosphere suitable for activating an inactivating-agent, the inactivating agent 18c supported by the virus inactivating filter set 18 is activated and the virus trapped in the filter set 18 is inactivated by the inactivating agent 18c. Time of heating operation for inactivating the virus in such a manner may be determined as needed, depending on a desired virus inactivation ratio.

By the condensed water generating and heating operations, the atmosphere for inactivating viruses in the inside space S of the air conditioner indoor unit 10 can be maintained for the required time so that the inactivating agent 18c supported by the virus inactivating filter set 18 is activated in the atmosphere and the virus trapped by the filter can be inactivated efficiently.

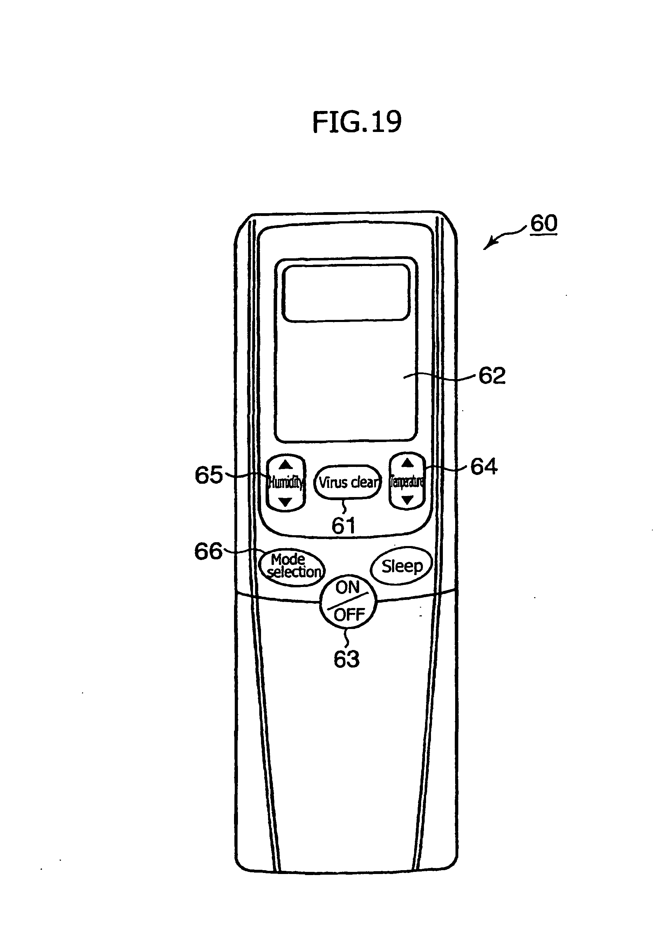

The above-described virus inactivating operation mode can be started with a single touch of a switch on a proper position of the operation panel or the like. For example, the operation can be started by pushing a virus clear button 61, which is provided with a remote controller 60 as illustrated in FIG. 19. When the virus clear button 61 is pushed, specific control signals for starting the virus inactivation operation mode may be generated. By pushing the virus clear button 61 on the remote controller 60, infrared control signals may be sent to a receiver of the air conditioner indoor unit 10 which is not illustrated. The remote controller 60 in FIG. 19 has, in addition to the virus clear button 61, a display means 62, start/stop button 63, temperature setting switch 64, humidity setting switch 65, and operation mode selection button 66.

The control signals are sent from the receiver to the controller (not illustrated) of the air conditioner unit 100. Receiving these signals, the controller carries out the above-described condensed water generating operation and heating operation in accordance with predetermined control steps, whereby the virus can be inactivated. The virus inactivating operation mode may be carried out in preference to the other operation modes when control signals are generated by pushing the virus clear button 61 and input to the controller. When the virus clear button 61 is pushed during the air-cooling or air-heating operation, the operation mode may be changed from the air-cooling or air-heating operation to the virus inactivating operation.

The virus inactivating operation mode can be stopped as needed during the operation. Control signals for interrupting the virus inactivating operation mode may be generated by pushing the virus clear button 61 again, or a new button exclusively used for interrupting the operation may be disposed on the remote controller 60. In this manner, the virus inactivating operation mode for starting operation or to interrupt operation can be selected by a single touch of the switch on the remote controller 60. Such a simple manner facilitates the virus inactivating operation. The virus inactivating operation mode may be set to work with a timer for the air-cooling or air-heating operation, which the air conditioner 100 conventionally has.

In the virus inactivating operation mode, a hot and humid atmosphere is formed as described above, but the time to get the desired target atmosphere may differ with the indoor or outdoor environment (temperature and humidity). The time necessary for generating a desired amount of the condensed water to reach a desired amount as a result of the condensed water generating operation or the time necessary for the condensed water to evaporate and attain a desired temperature and humidity may differ with the above-described environment. Conditions in condensed water generating operation are preferably controlled so as to facilitate formation of condensed water on the surfaces of the indoor heat exchangers 13, 14 and 15.

The specific examples of the operation conditions facilitating the generation of condensed water will next be described. A first specific example is to carry out operation while setting the opening degree of the electronic expansion valve 35 serving as a narrowing mechanism smaller compared with that upon ordinary air-cooling operation. This can increase a heat pumping capacity of the refrigerant and can lower the surface temperatures of the indoor heat exchangers 13, 14 and 15 so that the amount of condensed water appearing on the surfaces of the heat exchangers can be increased. In this case, the opening degree of the electronic expansion valve 35 may be controlled depending on the value (room temperature) detected by a room temperature detector disposed in the air conditioner indoor unit 10. The higher the room temperature, the smaller the opening degree of the electronic expansion valve 35.

A second specific example is to reduce the air flow passing through the indoor heat exchangers 13, 14 and 15. The reduction can be carried out by setting the rotation speed of the cross flow fan 17 below that upon ordinary air-cooling operation and thereby carrying out low-speed operation and decreasing the air flow sent from the fan. The operation can increase the amount of condensed water appearing on the surfaces of the heat exchangers, because the reduction in the heat pumping capacity of air leads to lowering in the surface temperatures of the indoor heat exchangers 13, 14 and 15.

A third specific example is to detect the outside air temperature and based thereon, to control the rotation speed of the outdoor fan 33 disposed in the air conditioner outdoor unit 30. In this case, when the outside air temperature is high, the amount of the refrigerant condensed by the outdoor heat exchanger 32 increases at a higher rotation speed of the outdoor fan 33. This causes a further increase in the refrigerant amount of the gas-liquid two-phase flow supplied to the indoor heat exchangers 13, 14 and 15. The surface temperatures of the indoor heat exchangers 13, 14 and 15 become lower, whereby the amount of the condensed water appearing on the surface of the heat exchanger can be increased.

The above-described first to third specific examples can be used either singly or in combination of two or more. Alternatively, all of these three examples may be used in combination.

The condensed water generating operation is not necessarily conducted prior to the condensed water heating operation. When the condensed water is generated by the ordinary air-cooling operation, the virus inactivating operation mode may be carried out by the condensed water heating operation following the air-cooling operation while positioning the air-cooling operation as the cooling operation for generating condensed water.

In the virus inactivating operation mode, the original object to inactivate viruses by activating the inactivating agent 18c can be attained by carrying out the above-described condensed water generating operation and heating operation. By adding the operations, which will be described later, before or after the condensed water generating operation, improvement in the efficiency of the virus inactivating operation and prolongation of the life of the inactivating agent 18c can be realized.

First, a virus trapping operation to be conducted prior to the condensed water generating operation will be described. This operation is to trap, in the virus inactivating filter set 18, viruses existing in the room by operating the cross flow fan 17 to suck the room air via the suction grille 11, causing the air to pass through the virus inactivating filter set 18 and then returning the air to the room via the air outlet 16. This operation aims to trap, in the virus inactivating filter set 18, viruses existing in the air so that only the passage of the indoor air through the virus inactivating filter set 18 is necessary and "fan mode" may be selected for circulation of the air. The indoor air can, of course, be circulated via the virus inactivating filter set 18 in the ordinary air-cooling·dehumidifying operation or air-heating operation so that a proper mode may be selected from the fan, cooling·dehumidifying and heating modes depending on the state in the room or user's preference.

When the air itself is circulated in the above-described manner, the air itself can pass through the filter body 18 but many of the viruses circulating with the air cannot pass through the filter and are trapped therein. If the virus trapping operation is continued for an adequate time, most of the viruses in the room are trapped in the virus inactivating filter set 18. The time for virus trapping operation can be determined in consideration of the size of the room, an expected amount of viruses existing therein, and virus trapping capacity of the virus inactivating filter set 18. When the virus inactivating operation mode is carried out while trapping many viruses in the filter, many viruses can be inactivated by a single virus inactivating operation. It is therefore possible to inactivate the viruses in the room efficiently and thereby keeping the room in a clean atmosphere with fewer viruses therein.

After completion of the heating operation in the virus inactivating mode, it is preferred to eliminate the hot and humid atmosphere in the inside space S as soon as possible. Particularly when the life of the inactivating agent 18c is taken into consideration, it is preferred to make the atmosphere cool and dry. In other words, the atmosphere bringing the activation level of the inactivating agent 18c back to those in the ordinary atmosphere level may be preferable in order to suppress the hydrolysis and self decomposition of the inactivating agent 18c which will otherwise occur by water remaining in the virus inactivating filter set 18. This enables suppression of a time-degration of the inactivating agent.

For example, in an air conditioner indoor unit equipped with a known ventilator (not illustrated) for discharging the indoor air to the outside, ventilation is performed for a certain time after the activated state of the inactivating agent is retained for a predetermined time and the condensed water heating operation is completed. In this ventilation operation, the hot and humid atmosphere existing in the inside space S can be discharged outside by actuating a ventilation fan (not illustrated), while closing the flap 21 to keep a semi-closed state of the inside space S in order to prevent deterioration, in the feeling brought by the use of the air conditioner, due to the flow of the hot and humid atmosphere into the room.

After the ventilation for a predetermined time to discharge the hot and humid air out of the room, air blowing by the cross flow fan 17 may be started in addition to the operation of the ventilation fan. In the case, the flap 21 is closed to maintain a semi-closed state of the inside space S and air flow occurs in the inside space S owing to the ventilation and air blowing, which enables dehumidification and drying of the virus inactivating filter set 18. The deterioration preventive operation may be conducted for an adequate period determined, depending on the capacity of the inside space S or the like.

When the control means of the air conditioner 100 performs the deterioration preventive operation mode comprising ventilation and air blowing operations after completion of the virus inactivation operation mode, the inside space S is released rapidly from the hot and humid environment. This can shorten activation time of the inactivating agent 18c so that the degradation of the inactivating agent 18 can be suppressed and the life can be prolonged. In short, the life of the virus inactivating filter set 18 until exchange can be prolonged.

In the above description, the air conditioner indoor unit is equipped with a ventilator. When the air conditioner indoor unit 10 has no ventilator, the ventilation operation may be replaced with the air blowing operation by the cross flow fan 17 in the semi-closed inside space S after completion of the condensed-water heating operation and the virus inactivating filter set 18 may be dried by the air flow caused by the operation.

In the above-described embodiments, both air blowing operation and ventilation operation are adopted for the deterioration preventive operation of the virus inactivating filter set when the unit has a ventilator, while air blowing operation is used singly as the deterioration preventive operation when the unit is not equipped with a ventilator. Even if the indoor unit is equipped with a ventilator, a deterioration preventive operation mode for removing moisture from the carrier of an inactivating agent may be selected from the single use of the air blowing operation or combined use of the air blowing operation and the ventilating operation, depending on the temperature and humidity for activating the inactivating agent.

In the above-described air conditioner indoor unit 10, the suction grille 11 is always open so that the inside space S is semi-closed in condensed water heating operation which needs closing of the flap 21.

An example of a modified air conditioner indoor unit 10A will next be described referring to FIG. 20. In the unit, the condensed water heating operation is conducted under the condition that the inside space S is closed. In the example, a switching means of a suction port such as suction port flap 12 is installed to the suction grille 11A so as to close the suction grille 11A as needed for example in condensed-water heating operation. In condensed water heating operation, the inside space S is hermetically sealed with the suction grille 11A and air outlet 16 being both closed by the flap, which can disturb leakage, to the outside, of the hot and humid atmosphere suitable for inactivating the virus.

When the condensed-water heating operation is conducted under the hermetically sealed condition, it may becomes easy to maintain the temperature and humidity in the inside space S because of no leakage of the atmosphere to the outside. This leads to an improvement in the efficiency to activate the inactivating agent 18c. In other words, the desired virus inactivating atmosphere can be formed in a shorter time. Further, an amount of energy or condensed water spent for maintaining the hot and humid atmosphere can be reduced, compared with the condensed-water heating operation under a semi-closed state.

In the condensed-water heating operation while hermetically sealing the inside space S, agitation of the air is preferably conducted by the cross flow fan 17. By the agitation, the hot and humid atmosphere in the hermetically sealed inside space S can be made substantially uniform.

This can activate the inactivating agent 18c uniformly all over the virus inactivating filter set 18. In other words, the virus inactivating agent 18c may act on the whole virus inactivating filter set 18 to inactivate viruses efficiently. In this manner, the filter set can work effectively.

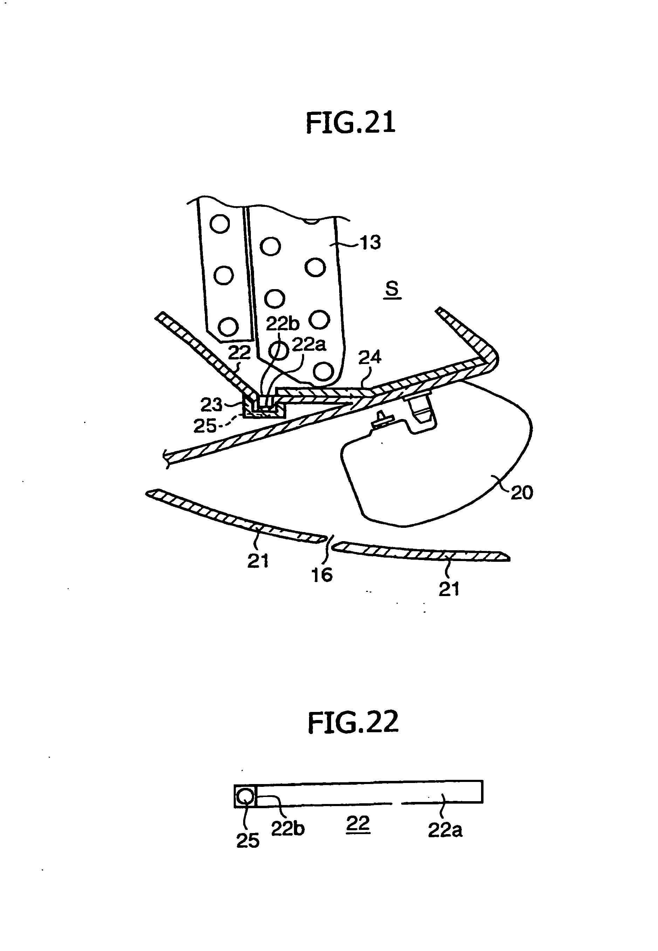

A second embodiment of an inactivating-agent activating means will next be described referring to FIGS. 21 and 22. The inactivating-agent activating means can heat and evaporate condensed water, which has been generated by the cooling operation of the indoor heat exchangers 13, 14 and 15 and accumulated in the drain pan 22, by a heating means such as electric heater 23 disposed at a proper place in the vicinity of the drain pan 22 to form a hot and humid atmosphere. In these diagrams, symbol indicated at 24 is a heat insulating material, while 25 is a drain hole made on the bottom surface of the drain pan 22.

A recess 22a is formed for accumulating therein the condensed water which has been generated by the ordinary air-cooling-dehumidifying operation or the above-described condensed water generating operation of the first embodiment and has dripped in the drain pan 22 from the surfaces of the heat exchangers. The recess 22a is preferably a trench formed on the bottom surface of the drain pan 22 and extending across the width direction of the air conditioner indoor unit 10, whereby the entire virus inactivating filter set 18 can be uniformly exposed to the vapor elevating substantially vertically from the recess. The recess 22a has a capacity of accumulating condensed water enough for raising the temperature and humidity of the inside space S to desired values and for maintaining the necessary heating operation time. The capacity can be defined by the height of an end plate 22b disposed in the vicinity of the drain hole 25 as well as the cross-sectional shape or length of the recess 22a.

The recess 22a is not limited to a trench extending across the width direction but various modification examples such as a plurality of separate recesses disposed at certain intervals across the width direction can be employed.

In the second embodiment, as in the heating operation in the first embodiment, the inside space S can be heated by turning on an electric heater 23 after it is semi-closed or hermetically closed. It is preferred to stop the operation of the cross flow fan 17 when the inside space S is semi-closed and to carry out the agitating operation when it is hermetically closed. In this case, the inactivating-agent activating means can be provided by adding the electric heater 23 as a heating means to an ordinary air conditioner indoor unit and giving a little change to the shape of the drain pan 22.

Similar to the operation or termination of the compressor 31 and outdoor fan 33 as illustrated in FIG. 19, operation of the electric heater 23 is preferably controlled as needed, for example, by energizing it intermittently so as to maintain necessary heating operation time.

As a result of retaining an internal air in the inside space of the air conditioning unit above described, the inside space S can be kept under hot and humid atmosphere suited for the activation of the inactivating agent. The inactivating agent 18 therefore can be activated to destroy viruses effectively, whereby these viruses can be inactivated. Various operations including the trapping operation and the ventilation operation conducted before or after the condensed water heating operation may be carried out as in the first embodiment.

As described above, the air conditioner indoor unit and the air conditioner equipped therewith according to the present disclosure have an inactivating-agent activating means capable of making the atmosphere suited for the activation of the inactivating agent 18c supported on the virus inactivating filter set 18 so that they can actively destroy and inactivate viruses, reduce the amount of viruses in the room and provide an environment with less infection possibility by viruses.

In these embodiments, air conditioner indoor units and air conditioners equipped therewith are described by giving some examples. Air conditioners for commercial spaces such as buildings are similar in the fundamental structure and function to the above-described air conditioner 114. Car air conditioners have a heating unit using cooling water for engine, but a filter set can be similarly attached to the air outlet for the car interior. The present invention can be applied to, as well as air conditioners, air cleaners, humidifiers and driers.

The virus inactivating agent used in the present invention can also be applied to masks, clothes for the medical service workers, coating materials for wall paper or wall surface, stretcher for carrying patients infected with a virus, and interior coating of capsules. The virus inactivating agent can also be used as a spray to be applied to medical wastes or facilities such as hospitals having patients accommodated therein. It can also be applied to a doormat, floor mat, carpet and automotive seat.

## Claims

1. A method for inactivating a virus, comprising a step of exposing a virus to a solution containing a virus inactivating agent,
wherein the virus inactivating agent is a virus inactivating agent for inactivating viruses in a liquid phase comprising as active ingredients a protein denaturant and the proteolytic enzyme pfu protease S.

2. The method according to Claim 1, wherein the protein denaturant is urea.

3. The method according to Claim 1, wherein the protein denaturant is a surfactant.

4. The method according to Claim 3, wherein the surfactant is sodium dodecyl sulfate (SDS).

5. The method according to any one of Claims 1 to 4, wherein the virus has an envelope.

6. The method according to any one of Claims 1 to 4, wherein the virus has not an envelope.

7. The method according to any one of Claims 1 to 6, wherein the virus inactivating agent has a bactericidal action.

8. The method according to any one of Claims 1 to 7, wherein the virus inactivating agent has a fungicidal action.

9. Use of a virus inactivating agent comprising as active ingredients a protein denaturant and the proteolytic enzyme pfu protease S for inactivating viruses in a liquid phase in a virus inactivating filter set, the virus inactivating filter set comprising a filter for trapping a virus and the virus inactivating agent adhered to the filter.

10. Use of a virus inactivating agent comprising as active ingredients a protein denaturant and the proteolytic enzyme pfu protease S for inactivating viruses in a liquid phase in an air conditioner unit, the air conditioner unit comprising: an air intake for introducing air therefrom; a heat exchanger for heating or cooling the air thus introduced from the air intake by heat exchange between the air and a refrigerant; an air outlet for releasing therefrom the air heat-exchanged by the heat exchanger; an air blower for promoting the release of the air from the air outlet; the virus inactivating filter set comprising a filter for trapping a virus and the said virus inactivating agent adhered to the filter, disposed in an inside space through which air flows; and a virus-inactivating-agent activating means for generating an atmosphere in the inside space suitable for the activation of the virus inactivating agent.

11. The use according to Claim 10, wherein the air conditioner unit further comprises a switching means for closing a part or the whole of an opening leading to the inside space so as to keep the inside space semi-closed or hermetically closed.

12. The use according to Claim 11, wherein the air suitable for the activation of the virus inactivating agent is agitated by the air blower in the hermetically closed space.

13. The use according to any one of Claims 10 to 12, wherein the virus-inactivating-agent activating means is for heating and evaporating water condensed in a cooling operation of the heat exchanger, in a heating operation of the heat exchanger conducted after the cooling operation.

14. The use according to any one of Claims 10 to 12, wherein the virus-inactivating-agent activating means is for heating and evaporating water condensed in a cooling operation of the heat exchanger and accumulated in a drain pan, by a heater.

15. A use according to Claim 13 or 14, wherein after the inside space is maintained hot and humid by the virus-inactivating-agent activating means, a deterioration preventive operation is conducted to remove water from the filter.

16. A use according to any one of Claims 10 to 15, wherein prior to the activation of the virus inactivating agent, a virus trapping operation is conducted by introducing air into the inside space, and causing the air to pass through the filter set.

17. A use according to any one of Claims 10 to 16, wherein the air conditioner unit further comprises an internal air retaining means for retaining the internal air in the inside space.

## Patentansprüche

1. Verfahren zur Inaktivierung eines Virus, umfassend Inkontaktbringen eines Virus mit einer Lösung, enthaltend ein Virus-Inaktivierungsmittel,
wobei das Virus-Inaktivierungsmittel ein Virus-Inaktivierungsmittel zur Inaktivierung von Viren in einer flüssigen Phase ist, umfassend als aktive Bestandteile ein Protein-Denaturierungsmittel und das proteolytische Enzym pfu-Protease S.

2. Verfahren gemäß Anspruch 1, wobei das Protein-Denaturierungsmittel Harnstoff ist.

3. Verfahren gemäß Anspruch 1, wobei das Protein-Denaturierungsmittel ein Tensid ist.

4. Verfahren gemäß Anspruch 3, wobei das Tensid Natriumdodecylsulfat (SDS) ist.

5. Verfahren gemäß einem der Ansprüche 1 bis 4, wobei das Virus eine Hülle besitzt.

6. Verfahren gemäß einem der Ansprüche 1 bis 4, wobei das Virus keine Hülle besitzt.

7. Verfahren gemäß einem der Ansprüche 1 bis 6, wobei das Virus-Inaktivierungsmittel eine bakterizide Wirkung hat.

8. Verfahren gemäß einem der Ansprüche 1 bis 7, wobei das Virus-Inaktivierungsmittel eine fungizide Wirkung hat.

9. Verwendung eines Virus-Inaktivierungsmittels, umfassend als aktive Bestandteile ein Protein-Denaturierungsmittel und das proteolytische Enzym pfu-Protease S zur Inaktivierung von Viren in einer flüssigen Phase in einem Virus-Inaktivierungsfiltersatz, wobei der Virus-Inaktivierungsfiltersatz einen Filter zum Abfangen eines Virus und das Virus-Inaktivierungsmittel, angehaftet an dem Filter, umfasst.

10. Verwendung eines Virus-Inaktivierungsmittels, umfassend als aktive Bestandteile ein Protein-Denaturierungsmittel und das proteolytische Enzym pfu-Protease S zur Inaktivierung von Viren in einer flüssigen Phase in einer Klimaanlage, wobei die Klimaanlage umfasst: einen Lufteinlass zum Einleiten von Luft daraus; einen Wärmetauscher zum Erwärmen oder Abkühlen der so von dem Lufteinlass eingeleiteten Luft durch Wärmeaustausch zwischen der Luft und einem Kühlmittel; einen Luftauslass zum Freisetzen der durch den Wärmetauscher erwärmten Luft daraus; ein Luftgebläse zum Fördern der Freisetzung der Luft aus dem Luftauslass; den Virus-Inaktivierungsfiltersatz, umfassend einen Filter zum Abfangen eines Virus und das genannte Virus-Inaktivierungsmittel, angehaftet an dem Filter, angeordnet in einem Innenraum, durch den Luft strömt; und ein Mittel zur Aktivierung des Virus-Inaktivierungsmittels zum Erzeugen einer Atmosphäre in dem Innenraum, die zur Aktivierung des Virus-Inaktivierungsmittels geeignet ist.

11. Verwendung gemäß Anspruch 10, wobei die Klimaanlage ferner eine Schaltereinrichtung zum Schließen eines Teils der Öffnung oder der gesamten Öffnung umfasst, um den Innenraum so halb verschlossen oder hermetisch verschlossen zu halten.

12. Verwendung gemäß Anspruch 11, wobei die Luft, die zur Aktivierung des Virus-Inaktivierungsmittels geeignet ist, durch das Luftgebläse in dem hermetisch verschlossenen Raum bewegt wird.

13. Verwendung gemäß einem der Ansprüche 10 bis 12, wobei das Mittel zur Aktivierung des Virus-Inaktivierungsmittels zum Erwärmen und Verdampfen von Wasser, welches in einem Kühlvorgang des Wärmetauschers kondensiert, in einem Erwärmungsvorgang des Wärmetauschers, der nach dem Kühlvorgang durchgeführt wird, vorgesehen ist.

14. Verwendung gemäß einem der Ansprüche 10 bis 12, wobei das Mittel zur Aktivierung des Virus-Inaktivierungsmittels zum Erwärmen und Verdampfen von Wasser, welches in einem Kühlvorgang des Wärmetauschers kondensiert und in einer Ablaufwanne angesammelt wird, durch eine Heizvorrichtung vorgesehen ist.

15. Verwendung gemäß Anspruch 13 oder 14, wobei, nachdem der Innenraum durch das Mittel zur Aktivierung des Virus-Inaktivierungsmittels heiß und feucht gehalten wird, ein Vorgang zur Prävention einer Störung durchgeführt wird, um Wasser aus dem Filter zu entfernen.

16. Verwendung gemäß einem der Ansprüche 10 bis 15, wobei, vor der Aktivierung des Virus-inaktivierenden Mittels, ein Virusabfangvorgang durchgeführt wird, in dem Luft in den Innenraum eingeströmt wird, und die Luft veranlasst wird, den Filtersatz zu durchströmen.

17. Verwendung gemäß einem der Ansprüche 10 bis 16, wobei die Klimaanlage ferner eine innere Luftverriegelung zum Halten der Innenluft in dem Innenraum umfasst.

## Revendications

1. Procédé d'inactivation d'un virus, comprenant une étape d'exposition d'un virus à une solution contenant un agent d'inactivation de virus,
dans lequel l'agent d'inactivation de virus est un agent d'inactivation de virus permettant d'inactiver des virus dans une phase liquide comprenant en tant que principes actifs un dénaturant de protéine et l'enzyme protéolytique protéase S de pfu.

2. Procédé selon la revendication 1, dans lequel le dénaturant de protéine est l'urée.

3. Procédé selon la revendication 1, dans lequel le dénaturant de protéine est un tensio-actif.

4. Procédé selon la revendication 3, dans lequel le tensio-actif est le dodécylsulfate de sodium (SDS).

5. Procédé selon l'une quelconque des revendications 1 à 4, dans lequel le virus a une enveloppe.

6. Procédé selon l'une quelconque des revendications 1 à 4, dans lequel le virus n'a pas d'enveloppe.

7. Procédé selon l'une quelconque des revendications 1 à 6, dans lequel l'agent d'inactivation de virus ayant une action bactéricide.

8. Procédé selon l'une quelconque des revendications 1 à 7, dans lequel l'agent d'inactivation de virus ayant une action fongicide.

9. Utilisation d'un agent d'inactivation de virus comprenant en tant que principes actifs un dénaturant de protéine et l'enzyme protéolytique protéase S de pfu pour inactiver des virus dans une phase liquide dans un ensemble de filtres d'inactivation de virus, l'ensemble de filtres d'inactivation de virus comprenant un filtre pour piéger un virus et l'agent d'inactivation de virus collé au filtre.

10. Utilisation d'un agent d'inactivation de virus comprenant en tant que principes actifs un dénaturant de protéine et l'enzyme protéolytique protéase S de pfu pour inactiver des virus dans une phase liquide dans une unité de conditionnement d'air, l'unité de conditionnement d'air comprenant : une entrée d'air pour introduire de l'air à partir de celle-ci ; un échangeur de chaleur pour chauffer ou refroidir l'air ainsi introduit à partir de l'entrée d'air par échange de chaleur entre l'air et un fluide frigorigène ; une sortie d'air pour libérer à partir de celle-ci l'air soumis à un échange de chaleur par l'échangeur de chaleur ; une soufflante d'air pour favoriser la libération de l'air à partir de la sortie d'air ; l'ensemble de filtres d'inactivation de virus comprenant un filtre pour piéger un virus et ledit agent d'inactivation de virus collé au filtre, disposé dans un espace intérieur à travers lequel l'air circule ; et un moyen d'activation d'agent d'inactivation de virus pour générer une atmosphère dans l'espace intérieur appropriée pour l'activation de l'agent d'inactivation de virus.

11. Utilisation selon la revendication 10, dans laquelle l'unité de conditionnement d'air comprend en outre un moyen de commutation pour fermer une partie ou la totalité d'une ouverture conduisant à l'espace intérieur de manière à maintenir l'espace intérieur fermé ou semi-fermé hermétiquement.

12. Utilisation selon la revendication 11, dans laquelle l'air approprié pour l'activation de l'agent d'inactivation de virus est agité par la soufflante d'air dans l'espace hermétiquement fermé.

13. Utilisation selon l'une quelconque des revendications 10 à 12, dans laquelle le moyen d'activation d'agent d'inactivation de virus est destiné au chauffage et à l'évaporation de l'eau condensée dans une opération de refroidissement de l'échangeur de chaleur, dans une opération de chauffage de l'échangeur de chaleur effectuée après l'opération de refroidissement.

14. Utilisation selon l'une quelconque des revendications 10 à 12, dans laquelle le moyen d'activation d'agent d'inactivation de virus est destiné au chauffage et à l'évaporation de l'eau condensée dans une opération de refroidissement de l'échangeur de chaleur et accumulée dans un bac de récupération, par un dispositif de chauffage.

15. Utilisation selon la revendication 13 ou 14, dans laquelle, après que l'espace intérieur est maintenu chaud et humide par le moyen d'activation d'agent d'inactivation de virus, une opération anti-dégradation est effectuée afin d'éliminer l'eau du filtre.

16. Utilisation selon l'une quelconque des revendications 10 à 15, dans laquelle avant l'activation de l'agent d'inactivation de virus, une opération de piégeage de virus est effectuée en introduisant de l'air dans l'espace intérieur, et en amenant l'air à passer à travers l'ensemble de filtres.

17. Utilisation selon l'une quelconque des revendications 10 à 16, dans laquelle l'unité de conditionnement d'air comprend en outre un moyen de retenue d'air interne pour retenir l'air interne dans l'espace intérieur.
